# EUROPEAN PATENT APPLICATION

(11) **EP 4 692 058 A1**
(43) Date of publication of application: **11.02.2026**
(21) Application number: 24780376.0
(22) Date of filing: 26.03.2024
(51) Int. Cl.: C07D 209/86, C07D 405/04, C07D 405/12, H10K 50/11, H10K 50/15, H10K 50/17, H10K 50/18, H10K 59/10, H10K 85/60

(54) **ARYLAMINE COMPOUND, ORGANIC ELECTROLUMINESCENT ELEMENT, AND ELECTRONIC DEVICE**

(30) Priority: 29.03.2023 JP 2023052571
(71) Applicant: Hodogaya Chemical Co., Ltd., Tokyo, 105-0021 (JP)
(72) Inventor: KASE, Kouki, Tokyo 105-0021 (JP); LIM, Jae-Geon, Tokyo 105-0021 (JP); CHOI, Young-Tae, Tokyo 105-0021 (JP); SHIN, Heung-Seob, Tokyo 105-0021 (JP); IZUMIDA, Junichi, Tokyo 105-0021 (JP); HAYASHI, Shuichi, Tokyo 105-0021 (JP)
(74) Representative: Forstmeyer, Dietmar
(86) International application number: PCT/JP2024/012082
(87) International publication number: WO 2024/204268

(57) **Abstract**

An object of the present invention is to provide an organic compound with excellent properties, including excellent hole injection and transport performance, electron blocking capability, and high stability in a thin film state, as a material for a highly efficient and highly durable organic EL element. Another object of the present invention is to provide a highly efficient and highly durable organic EL element using this compound. Specific arylamine compounds of the present invention have excellent heat resistance and good hole transport capability. Organic EL elements in which these compounds were used in a hole transport layer, an electron blocking layer, a light emitting layer, and a hole injection layer exhibited good element properties.

## Description

### Technical Field

The present invention relates to a compound suitable for use in an organic electroluminescent element (hereinafter abbreviated as an "organic EL element"), which is a self-luminescent element favorably used in various display devices, and to an element. More particularly, the present invention relates to an arylamine compound and an organic EL element using this compound.

### Background Art

Since organic EL elements are self-luminescent elements, they are brighter, have superior display viewability, and can provide a clearer display, compared with liquid crystal elements. For these reasons, active studies have been carried out on organic EL elements.

In 1987, C. W. Tang et al. of Eastman Kodak Company produced a practical organic EL element made of an organic material, by developing an element having a stacked layer structure in which various functions were assigned to different materials. They achieved a high luminance of 1000 cd/m² or higher at a voltage of 10 V or less by stacking a layer of a fluorescent body capable of transporting electrons and a layer of an organic substance capable of transporting holes, and injecting both charges into the fluorescent body layer to cause the layer to emit light (see Patent Literature 1 and Patent Literature 2, for example).

Many improvements have been heretofore made to organic EL elements to put them to practical use, and high efficiency and durability have been achieved by electroluminescent elements in which various functions of the stacked layer structure are subdivided even further, and an anode, a hole injection layer, a hole transport layer, a light emitting layer, an electron transport layer, an electron injection layer, and a cathode are sequentially provided on a substrate (see Non-Patent Literature 1, for example).

To further increase light emission efficiency, attempts have been made to utilize triplet excitons, and the utilization of phosphorescent light emitting compounds has been investigated (see Non-Patent Literature 2, for example). Furthermore, elements that utilize light emission based on thermally activated delayed fluorescence (TADF) have also been developed. In 2011, Adachi et al. from Kyushu University realized an external quantum efficiency of 5.3% with an element that uses a thermally activated delayed fluorescence material (see Non-Patent Literature 3, for example).

The light emitting layer can also be prepared by doping a charge transporting compound, generally called a host material, with a fluorescent compound, a phosphorescent light emitting compound, or a material that radiates delayed fluorescence. As stated in the non-patent literature mentioned above, the selection of organic materials in an organic EL element greatly affects the properties of that element, such as efficiency and durability (see Non-Patent Literature 1, Non-Patent Literature 2, and Non-Patent Literature 3, for example).

In an organic EL element, in which the charges injected from both electrodes recombine in the light emitting layer, thereby producing light emission, how efficiently the charges of both the holes and the electrons are passed on to the light emitting layer is important, and the element needs to have an excellent carrier balance. Therefore, high light emission efficiency can be achieved by increasing the probability of holes and electrons recombining in the light emitting layer by using a material with properties that improve hole injectability, that is, the ability to supply holes injected from the anode to the light emitting layer, and electron blockability, that is, the ability to block electrons injected from the cathode, and furthermore, by confining excitons generated in the light emitting layer. For this purpose, the functions to be fulfilled by the hole transport material are important, and a hole transport material having high hole injectability, high hole mobility, high electron blockability, and high durability against electrons has been in demand.

Moreover, with regard to element lifespan, the heat resistance and amorphousness of the materials are also important. A material with low heat resistance thermally decomposes due to heat produced during element driving, even at low temperatures, leading to material deterioration. A material with low amorphousness causes crystallization of a thin film to occur even in a short period of time, leading to element deterioration. Thus, the materials to be used are required to have high heat resistance and good amorphousness.

Examples of conventional hole transport materials used in organic EL elements include N,N'-diphenyl-N,N'-di(α-naphthyl)benzidine (NPD) and various aromatic amine derivatives (see Patent Literature 1 and Patent Literature 2, for example). However, although NPD has good hole transport capability, its glass transition point (Tg), which is an indicator of heat resistance, is as low as 96°C, and therefore, under high-temperature conditions, the element properties degrade due to crystallization (see Non-Patent Literature 4, for example).

Among the aromatic amine derivatives disclosed in the patent literature mentioned above, there are compounds with an excellent hole mobility of 10⁻³ cm²/Vs or higher (see Patent Literature 1 and Patent Literature 2, for example), but the electron blockability of these compounds is insufficient, which allows some electrons to pass through the light emitting layer, and therefore, for example, no increase in light emission efficiency can be expected. Thus, to further increase efficiency, materials having higher electron blockability, higher stability in a thin film state, and higher heat resistance are needed. Although aromatic amine derivatives with high durability have also been reported (see Patent Literature 3, for example), these aromatic amine derivatives were used as charge transport materials for electrophotographic photoreceptors, and there were no examples of their use in organic EL elements.

To address these issues, substituted carbazole structures and arylamine compounds have been proposed as compounds with improved properties, such as heat resistance and hole injectability (see Patent Literature 4 and Patent Literature 5, for example). However, even though the element lifespan, light emission efficiency, and other properties of elements using these compounds in a hole injection layer or a hole transport layer have been improved, the results are still insufficient. Therefore, there is a demand for a further decrease in driving voltage, a further increase in light emission efficiency, and a further increase in element lifespan.

### Citation List

### Patent Literature

Patent Literature 1: U.S. Patent No. 5792557
Patent Literature 2: U.S. Patent No. 5639914
Patent Literature 3: U.S. Patent No. 7759030
Patent Literature 4: JP 2009-076817A
Patent Literature 5: U.S. Patent No. 10818844
Patent Literature 6: European Patent No. 2684932
Patent Literature 7: U.S. Patent No. 1046895
Patent Literature 8: Korean Patent No. 10-2107875

### Non-Patent Literature

Non-Patent Literature 1: Proceedings of the 9th Meeting of the Japan Society of Applied Physics, pp. 55-61 (2001)
Non-Patent Literature 2: Proceedings of the 9th Meeting of the Japan Society of Applied Physics, pp. 23-31 (2001)
Non-Patent Literature 3: Appl. Phys. Let., 98, 083302 (2011)
Non-Patent Literature 4: Proceedings of the 3rd Meeting of the Japan OLED Forum, pp. 13-14 (2006)
Non-Patent Literature 5: Chem. Rev., 116, 12564-12649 (2016)

### Summary of Invention

An object of the present invention is to provide, as a material for highly efficient and highly durable organic EL elements, a material for organic EL elements that has (1) excellent hole injection and transport performance, (2) electron blocking capability, (3) high stability in a thin film state, and (4) excellent durability.

Another object of the present invention is to provide an organic EL element that has (1) high light emission efficiency and power efficiency, (2) a low light-emission start voltage and a low actual driving voltage, and (3) a long lifespan, by using the material of the present invention.

To achieve the above-described objects, the inventors of the present invention focused on the excellent hole injection and transport capabilities, stability in a thin film state, and durability of arylamine compounds, and were able to markedly improve the material properties by optimizing the substitution position of carbazolyl groups and substituents. Also, in organic EL elements, performance was improved in terms of light emission efficiency and power efficiency, which enabled the suppression of the light-emission start voltage and the actual driving voltage, thereby realizing a longer lifespan than conventional lifespans. Thus, the present invention was achieved.

That is, according to the present invention, an arylamine compound as well as an organic EL element and an electronic device that use the arylamine compound are provided as described below.

1) An arylamine compound represented by the general formula (A) below.

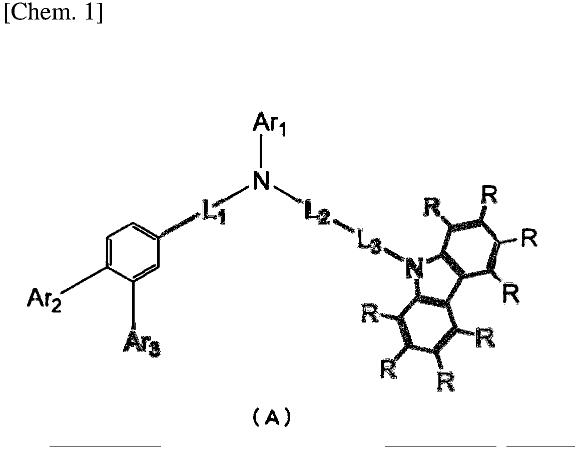

In the formula (A), Ar₁, Ar₂, and Ar₃ each independently represent a substituted or unsubstituted aromatic hydrocarbon group, a substituted or unsubstituted aromatic heterocyclic group, or a substituted or unsubstituted fused polycyclic aromatic group,
L₁, L₂, and L₃ each independently represent a single bond, a substituted or unsubstituted divalent aromatic hydrocarbon group, a substituted or unsubstituted divalent aromatic heterocyclic group, or a substituted or unsubstituted divalent fused polycyclic aromatic group, and
each R independently represents a hydrogen atom, a heavy hydrogen atom, a substituted or unsubstituted aromatic hydrocarbon group, a substituted or unsubstituted aromatic heterocyclic group, or a substituted or unsubstituted fused polycyclic aromatic group.

2) The arylamine compound as set forth in clause 1), wherein each R in the general formula (A) independently represents a hydrogen atom, a heavy hydrogen atom, or a substituted or unsubstituted phenyl group.

3) The arylamine compound as set forth in clause 1), wherein L₁, L₂, and L₃ each independently represent a single bond, an unsubstituted divalent aromatic hydrocarbon group, an unsubstituted divalent aromatic heterocyclic group, or an unsubstituted divalent fused polycyclic aromatic group.

4) The arylamine compound as set forth in clause 3), wherein L₁, L₂, and L₃ in the general formula (A) each independently represent a single bond, an unsubstituted divalent phenylene group, an unsubstituted divalent naphthylene group, or an unsubstituted divalent dibenzofuranyl group.

5) The arylamine compound as set forth in clause 4), wherein L₃ in the general formula (A) represents an unsubstituted 1,2-phenylene group, an unsubstituted 1,3-phenylene group, an unsubstituted 1,7-dibenzofuranyl group, or an unsubstituted 2,7-dibenzofuranyl group.

6) The arylamine compound as set forth in clause 4), wherein L₁ and L₂ in the general formula (A) each independently represent a single bond or an unsubstituted 1,4-phenylene group.

7) The arylamine compound as set forth in clause 1), wherein at least one of Ar₁, Ar₂, and Ar₃ in the general formula (A) represents a substituted or unsubstituted phenyl group.

8) An organic EL element comprising:
a pair of electrodes; and
an organic layer sandwiched between the electrodes,
wherein the organic layer contains the arylamine compound as set forth in any one of clauses 1) to 7).

9) The organic EL element as set forth in clause 8), wherein the organic layer is a hole transport layer, an electron blocking layer, a hole injection layer, or a light emitting layer.

10) An electronic device comprising an element comprising:
a pair of electrodes; and
an organic layer sandwiched between the electrodes,
wherein the organic layer contains the arylamine compound as set forth in any one of clauses 1) to 7).

The "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by Ar₁ to Ar₃ or R in the general formula (A) may specifically refer to a group selected from aryl groups having 6 to 30 carbon atoms and heteroaryl groups having 2 to 20 carbon atoms, such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzooxazolyl group, a benzothiazolyl group, an oxazolopyridyl group, an oxazolopyrazyl group, a quinoxalinyl group, a quinazolinyl group, a benzimidazolyl group, a pyrazolyl group, a dibenzofuranyl group, a dibenzothienyl group, a naphthyridinyl group, a phenanthronyl group an acridinyl group, and a carbolinyl group.

The "divalent aromatic hydrocarbon group", the "divalent aromatic heterocyclic group", or the "divalent fused polycyclic aromatic group" in the "substituted or unsubstituted divalent aromatic hydrocarbon group", the "substituted or unsubstituted divalent aromatic heterocyclic group", or the "substituted or unsubstituted divalent fused polycyclic aromatic group" represented by L₁ to L₃ in the general formula (A) may refer to a divalent group obtained by removing one hydrogen atom from the "aromatic hydrocarbon group", the "aromatic heterocyclic group", or the "fused polycyclic aromatic group" represented by Ar₁ to Ar₃ or R in the general formula (A), for example, a divalent group obtained by removing one hydrogen atom from any of the groups listed above as specific examples.

The "substituent" in the "substituted aromatic hydrocarbon group", the "substituted aromatic heterocyclic group", or the "substituted fused polycyclic aromatic group" represented by Ar₁ to Ar₃, L₁ to L₃, or R in the general formula (A) may specifically refer to a heavy hydrogen atom, a cyano group, or a nitro group; a halogen atom such as a fluorine atom, a chlorine atom, a bromine atom, or an iodine atom; a silyl group such as a trimethylsilyl group or a triphenylsilyl group; a linear or branched alkyl group having 1 to 6 carbon atoms such as a methyl group, an ethyl group, or a propyl group; a linear or branched alkyloxy group having 1 to 6 carbon atoms such as a methyloxy group, an ethyloxy group, or a propyloxy group; an alkenyl group such as a vinyl group or an allyl group; an aryloxy group such as a phenyloxy group or a tolyloxy group; an arylalkyloxy group such as a benzyloxy group or a phenethyloxy group; an aromatic hydrocarbon group or a fused polycyclic aromatic group such as a phenyl group, a biphenyl group, a terphenyl group, a naphthyl group, an anthracenyl group, a phenanthrenyl group, a fluorenyl group, a spirobifluorenyl group, an indenyl group, a pyrenyl group, a perylenyl group, a fluoranthenyl group, or a triphenylenyl group; an aromatic heterocyclic group such as a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, or a carbolinyl group; or the like, and these substituents may be further substituted with the above-listed substituents. Furthermore, such a substituent and a benzene ring substituted with the substituent, or a plurality of substituents on the same benzene ring, may be bonded to each other via a single bond, a substituted or unsubstituted methylene group, an oxygen atom, or a sulfur atom to form a ring.

In the general formula (A), L₁ preferably represents a single bond or an unsubstituted 1,4-phenylene group, and more preferably a single bond.

In the general formula (A), L₂ preferably represents a single bond or an unsubstituted 1,4-phenylene group.

In the general formula (A), L₃ preferably represents an unsubstituted 1,2-phenylene group, a 1,3-phenylene group, or a 1,7-benzofuranyl group, and more preferably a 1,2-phenylene group or a 1,3-phenylene group.

In the general formula (A), preferably, L₂ represents an unsubstituted 1,4-phenylene group, and L₃ represents a 1,2-phenylene group or a 1,3-phenylene group. Alternatively, preferably, L₂ represents a single bond, and L₃ represents a 1,7-benzofuranyl group.

In the general formula (A), Ar₁ preferably represents a substituted phenyl group, a substituted or unsubstituted biphenylyl group, or a benzofuranyl group, and more preferably a substituted or unsubstituted 4-[1,1']biphenylyl group. In addition, the substituent of the substituted phenyl group is preferably a naphthyl group.

In the general formula (A), Ar₂ preferably represents an unsubstituted phenyl group or an unsubstituted 4-[1,1']biphenylyl group.

In the general formula (A), Ar₃ preferably represents an unsubstituted phenyl group or an unsubstituted 4-[1,1']biphenylyl group, and more preferably an unsubstituted phenyl group.

In the general formula (A), preferably, all of R represent hydrogen atoms.

The arylamine compound represented by the general formula (A) that is suitable for use in the organic EL element of the present invention has excellent hole injection and transport capabilities, stability in a thin film state, and durability, and is therefore preferably used as a material constituting a hole injection layer, a hole transport layer, an electron blocking layer, or a light emitting layer of the organic EL element, and more preferably as a material constituting the hole transport layer or the electron blocking layer.

In addition, in an electronic device using an element that includes a pair of electrodes and at least one organic layer sandwiched therebetween, the arylamine compound represented by the general formula (A) of the present invention is also preferably used as a material constituting the organic layer.

The arylamine compound represented by the general formula (A) of the present invention has (1) better hole injection properties; (2) higher hole mobility; (3) superior electron blocking capability; (4) higher electron tolerance; (5) higher stability in a thin film state; (6) superior heat resistance; and other properties, compared with conventional hole transport materials.

An organic EL element with a hole injection layer and/or a hole transport layer prepared using the arylamine compound represented by the general formula (A) of the present invention as a hole injection material and/or a hole transport material has increased hole transport efficiency to a light emitting layer, resulting in increased light emission efficiency and reduced driving voltage, which can enhance the durability of the element. Consequently, the following element properties can be achieved: (1) high light emission efficiency, (2) a low light-emission start voltage, (3) a low actual driving voltage, and (4) a long lifespan.

The arylamine compound represented by the general formula (A) of the present invention has excellent electron blocking capability and high electron tolerance, is stable even in a thin film state, and has the characteristic of confining excitons generated in the light emitting layer. Thus, an organic EL element with an electron blocking layer prepared using this compound as an electron blocking material has high light emission efficiency, reduced driving voltage, and improved current tolerance, due to the increased probability of recombination between holes and electrons and the suppression of thermal deactivation. Consequently, the maximum light emission luminance is increased.

The arylamine compound represented by the general formula (A) of the present invention has excellent hole transportability and a wide band gap. Thus, an organic EL element with a light emitting layer prepared using this compound as a host material has reduced driving voltage and improved light emission efficiency when the light emitting layer is formed by incorporating a fluorescent emitter, a phosphorescent emitter, or a delayed fluorescent emitter, which is called a dopant.

Accordingly, the arylamine compound represented by the general formula (A) of the present invention is useful as a material constituting a hole injection layer, a hole transport layer, an electron blocking layer, or a light emitting layer of an organic EL element and can improve the light emission efficiency, driving voltage, and durability of conventional organic EL elements.

Furthermore, the arylamine compound represented by the general formula (A) of the present invention can not only be applied to organic EL elements but also be used in the field of electronic apparatuses such as electrophotographic photoreceptors, image sensors, photoelectric conversion elements, and solar cells.

### Brief Description of Drawings

[Fig. 1] The figure shows the structures of Compounds (1) to (18) as example arylamine compounds represented by the general formula (A).
[Fig. 2] The figure shows the structures of Compounds (19) to (33) as example arylamine compounds represented by the general formula (A).
[Fig. 3] The figure shows the structures of Compounds (34) to (48) as example arylamine compounds represented by the general formula (A).
[Fig. 4] The figure shows the structures of Compounds (49) to (63) as example arylamine compounds represented by the general formula (A).
[Fig. 5] The figure shows the structures of Compounds (64) to (78) as example arylamine compounds represented by the general formula (A).
[Fig. 6] The figure shows the structures of Compounds (79) to (90) as example arylamine compounds represented by the general formula (A).
[Fig. 7] The figure shows the structures of Compounds (91) to (102) as example arylamine compounds represented by the general formula (A).
[Fig. 8] The figure shows the structures of Compounds (103) to (120) as example arylamine compounds represented by the general formula (A).
[Fig. 9] The figure shows the structures of Compounds (121) to (135) as example arylamine compounds represented by the general formula (A).
[Fig. 10] The figure shows the structures of Compounds (136) to (150) as example arylamine compounds represented by the general formula (A).
[Fig. 11] The figure shows the structures of Compounds (151) to (162) as example arylamine compounds represented by the general formula (A).
[Fig. 12] The figure shows the structures of Compounds (163) to (174) as example arylamine compounds represented by the general formula (A).
[Fig. 13] The figure shows the structures of Compounds (175) to (186) as example arylamine compounds represented by the general formula (A).
[Fig. 14] The figure shows the structures of Compounds (187) to (198) as example arylamine compounds represented by the general formula (A).
[Fig. 15] The figure shows the structures of Compounds (199) to (210) as example arylamine compounds represented by the general formula (A).
[Fig. 16] The figure shows the structures of Compounds (211) to (222) as example arylamine compounds represented by the general formula (A).
[Fig. 17] The figure shows the structures of Compounds (223) to (234) as example arylamine compounds represented by the general formula (A).
[Fig. 18] The figure shows the structures of Compounds (235) to (246) as example arylamine compounds represented by the general formula (A).
[Fig. 19] The figure shows the structures of Compounds (247) to (258) as example arylamine compounds represented by the general formula (A).
[Fig. 20] The figure shows an example of the structure of an organic EL element of the present invention.

### Description of Embodiments

Although arylamine compounds represented by the general formula (A) of the present invention are novel compounds, these compounds can be synthesized using a method that is known per se. For example, a compound of interest can be synthesized through a coupling reaction with a known palladium catalyst or the like (see Non-Patent Literature 5, for example).

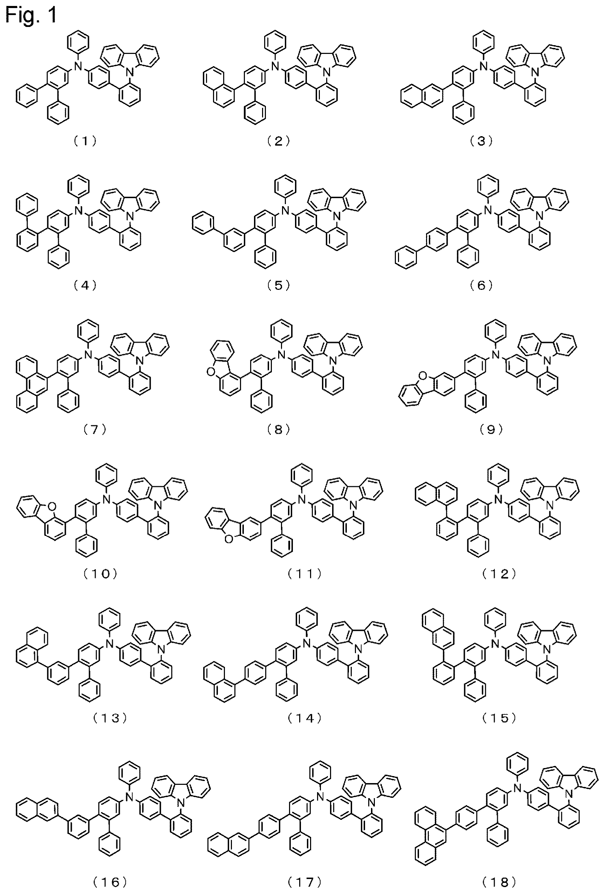

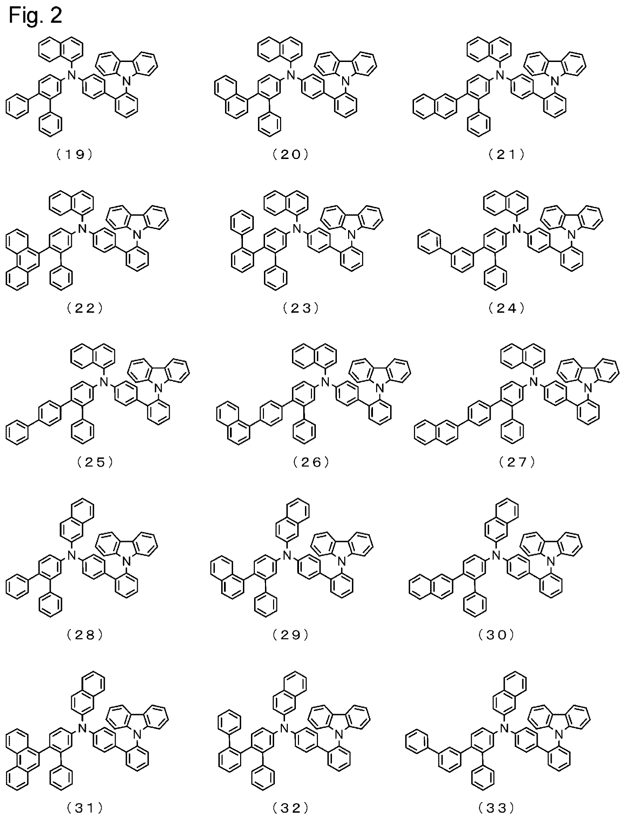

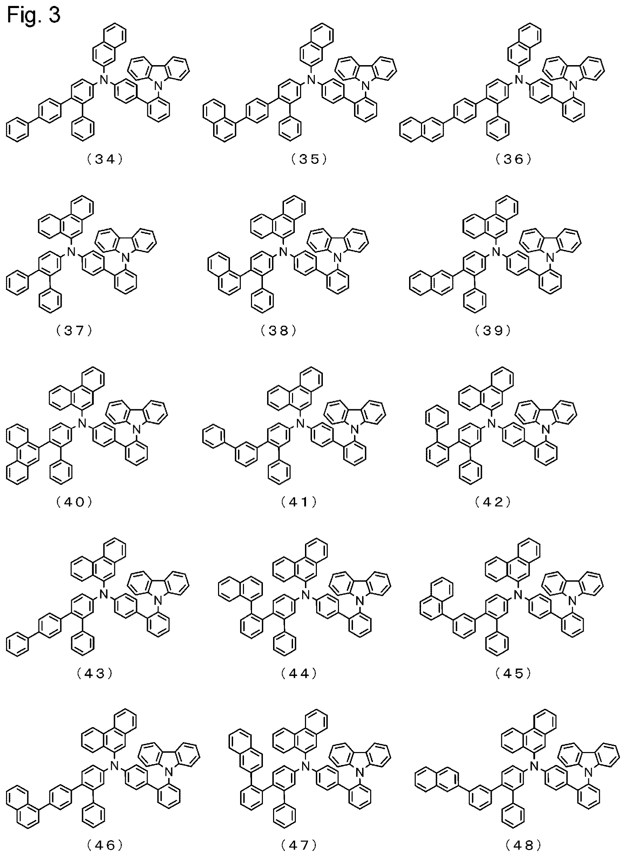

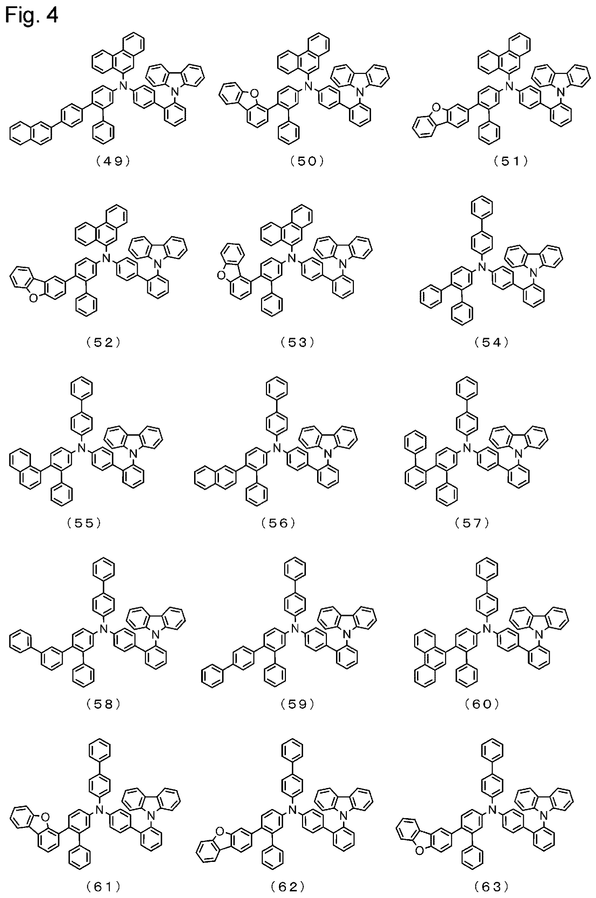

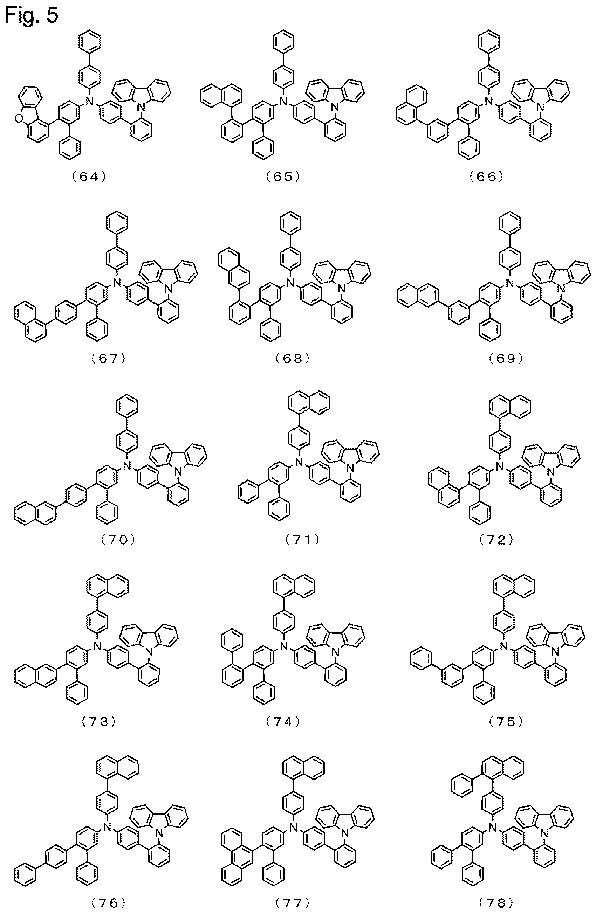

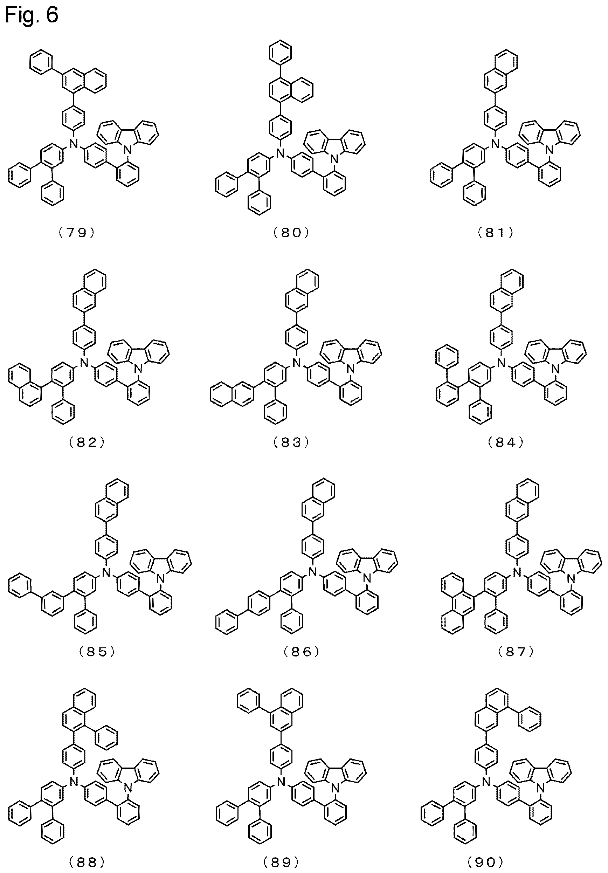

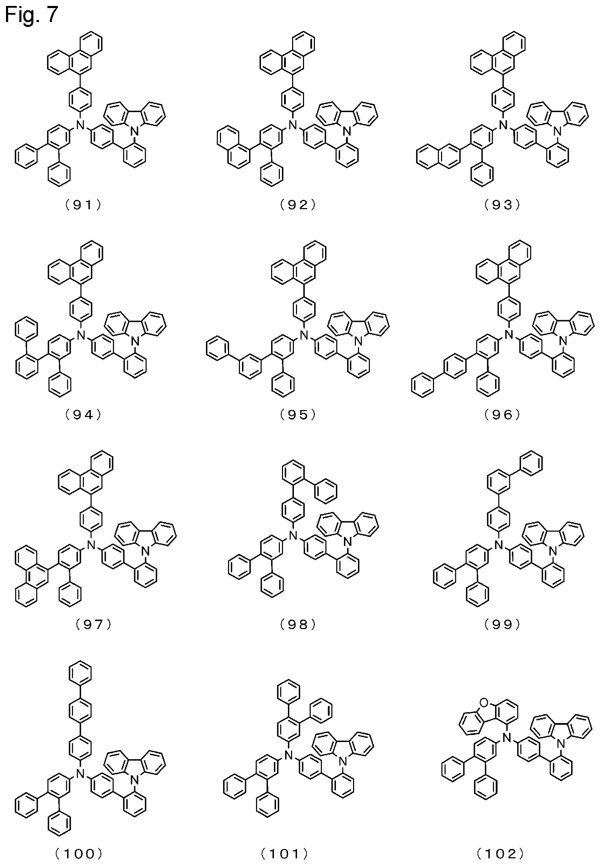

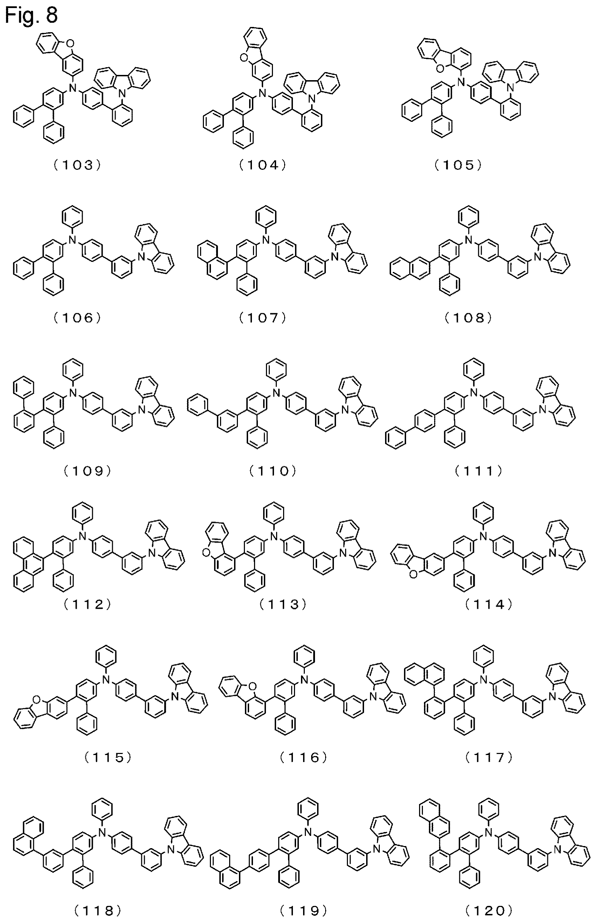

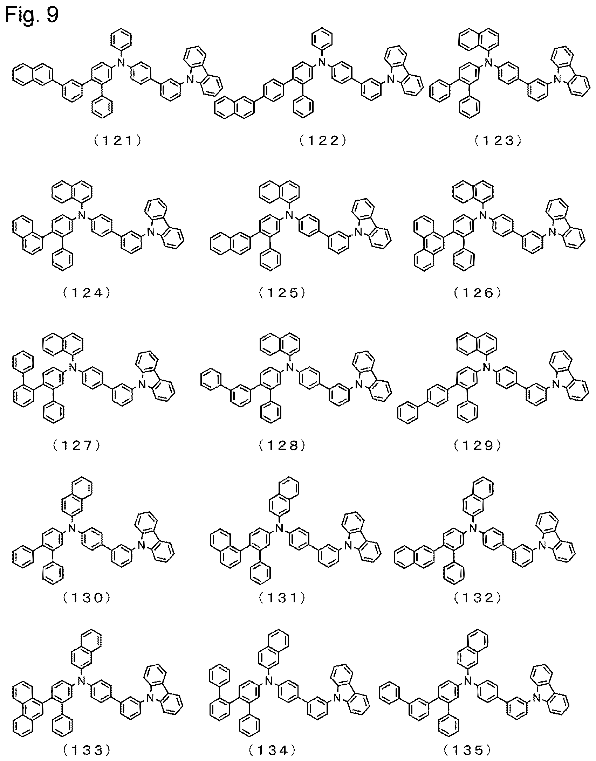

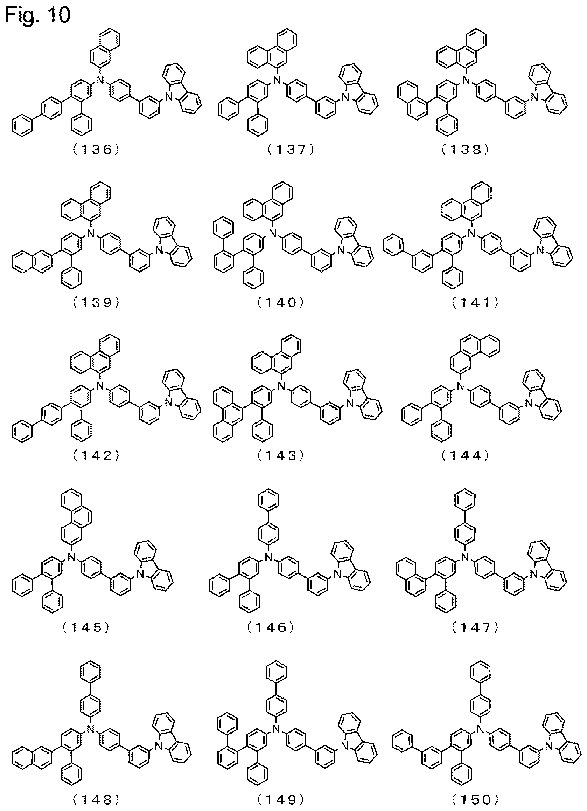

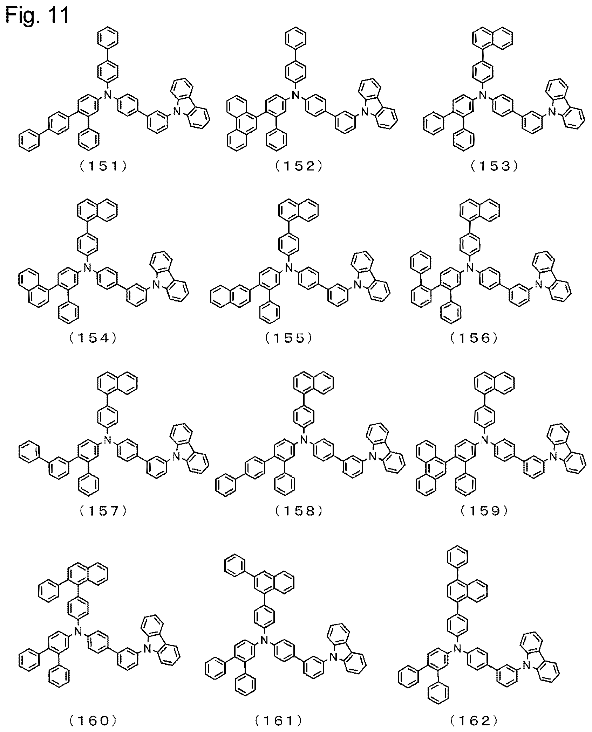

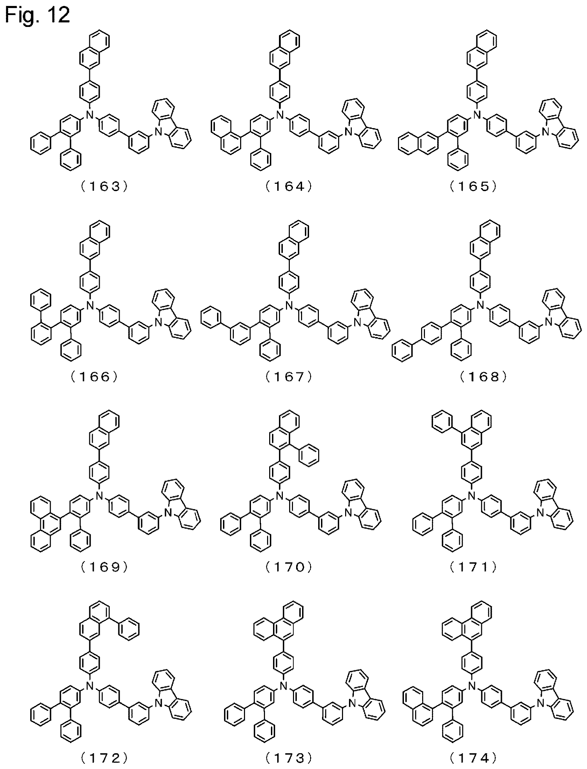

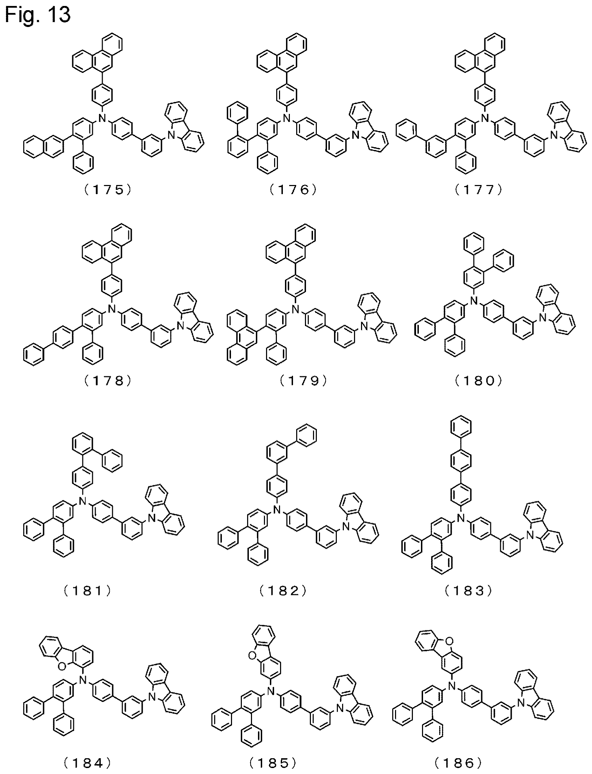

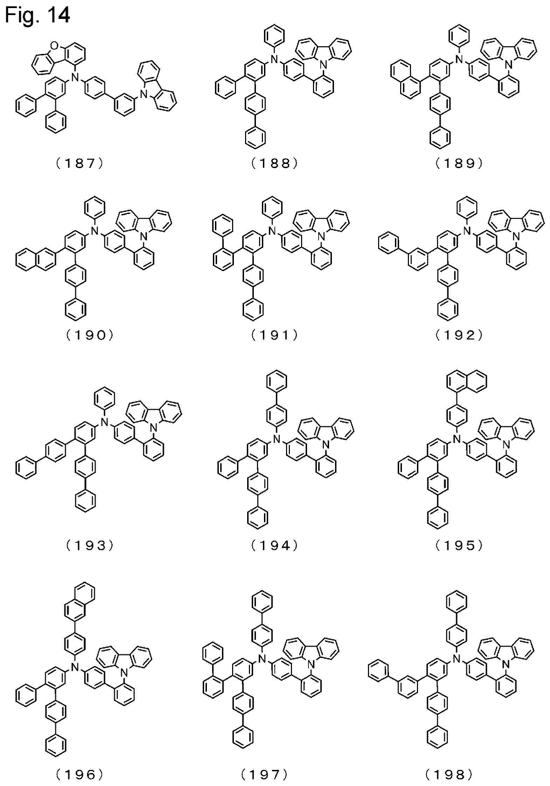

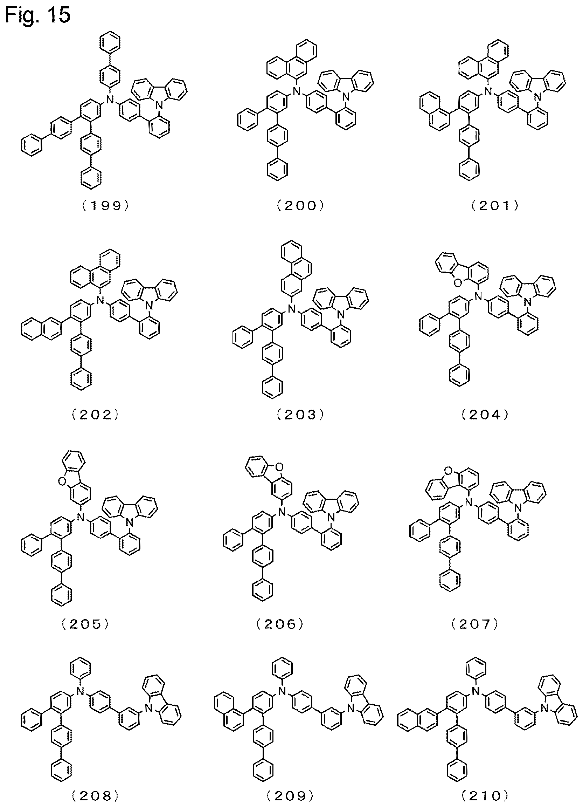

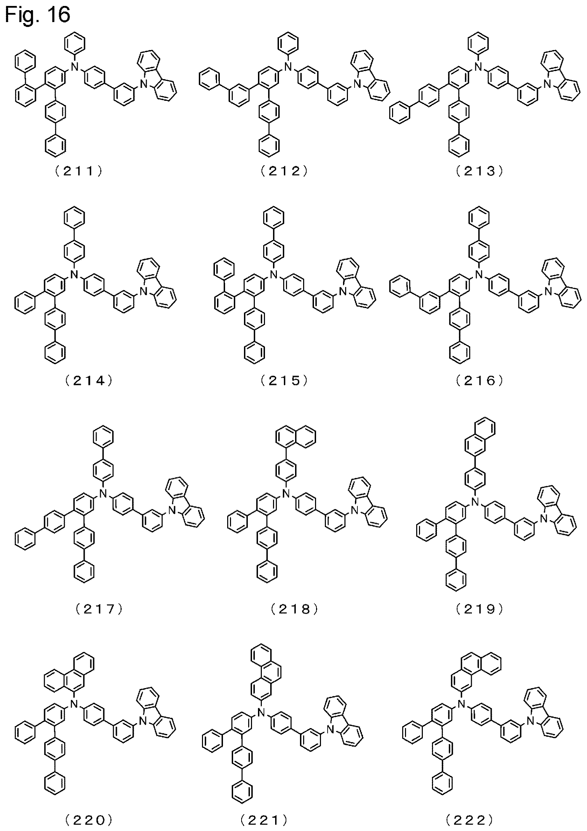

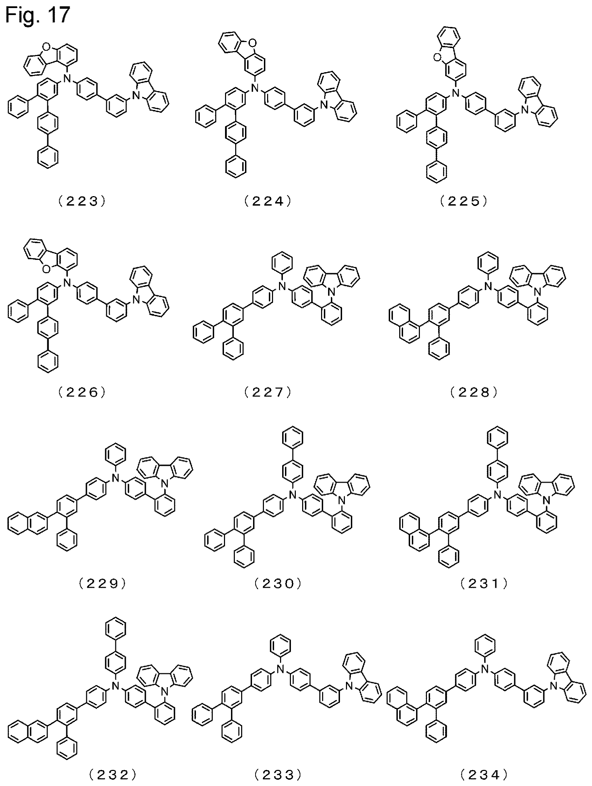

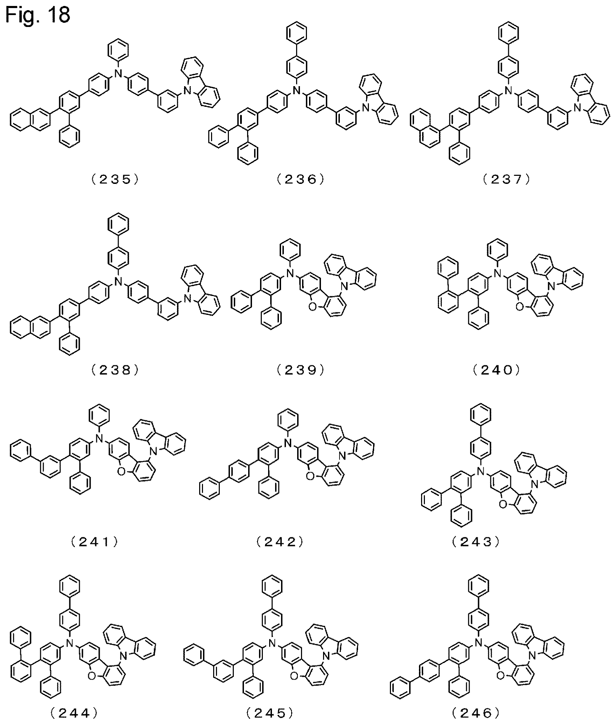

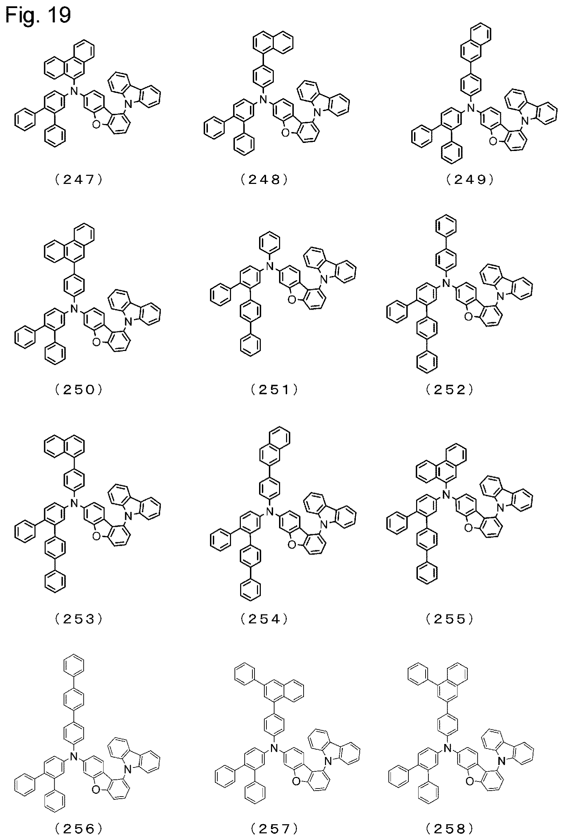

Figs. 1 to 19 show specific examples of preferred compounds among arylamine compounds represented by the general formula (A) above, which are suitable for use in an organic EL element of the present invention. However, the present invention is not limited to these compounds.

An arylamine compound represented by the general formula (A) of the present invention can be purified using known methods such as purification through column chromatography, adsorption purification using silica gel, activated carbon, activated clay, or the like, recrystallization or crystallization using a solvent, and sublimation purification. Final purification is preferably performed by sublimation. Compounds were identified by NMR analysis.

As other compounds for use in an organic EL element of the present invention, those purified by performing purification through column chromatography, adsorption purification using silica gel, activated carbon, activated clay, or the like, recrystallization or crystallization using a solvent, or the like and then finally purified by sublimation were used.

The melting point, glass transition point (Tg), and work function were measured as physical property values of arylamine compounds represented by the general formula (A) of the present invention. The melting point is an indicator of vapor deposition properties, the glass transition point (Tg) is an indicator of stability in a thin film state, and the work function is an indicator of hole injectability and hole transportability or electron blockability.

The melting point and the glass transition point (Tg) can be obtained, for example, by performing measurement on a compound in powder form using a high-sensitivity differential scanning calorimeter (DSC3100SA manufactured by Bruker AXS K.K.).

The work function can be obtained, for example, by forming a 100-nm thin film on an ITO substrate and performing measurement using an ionization potential measuring device (PYS-202 manufactured by Sumitomo Heavy Industries, Ltd.).

The organic EL element of the present invention may have a structure in which an anode, a hole injection layer, a hole transport layer, a light emitting layer, an electron transport layer, an electron injection layer, a cathode, and a capping layer are sequentially provided on a substrate. Alternatively, the organic EL element of the present invention may have a structure in which an electron blocking layer is further provided between the hole transport layer and the light emitting layer, or a structure in which a hole blocking layer is further provided between the light emitting layer and the electron transport layer. In these multilayer structures, a single organic layer may perform the functions of several layers, and, for example, it is possible to adopt a configuration in which a single organic layer serves as both the hole injection layer and the hole transport layer, a configuration in which a single organic layer serves as both the electron injection layer and the electron transport layer, and the like. Furthermore, two or more organic layers having the same function may be stacked, and it is possible to adopt a configuration in which two hole transport layers are stacked, a configuration in which two light emitting layers are stacked, a configuration in which two electron transport layers are stacked, and the like.

For the anode of the organic EL element of the present invention, an electrode material with a high work function, such as ITO or gold, is used.

As a material for the hole injection layer and the hole transport layer of the organic EL element of the present invention, in addition to the arylamine compound of the present invention being used, benzidine derivatives such as N,N'-diphenyl-N,N'-di(m-tolyl)benzidine (TPD), N,N'-diphenyl-N,N'-di(α-naphthyl)benzidine (NPD), and N,N,N',N'-tetrabiphenylyl benzidine; 1,1-bis[(di-4-tolylamino)phenyl]cyclohexane (TAPC); arylamine compounds with a structure containing two or more triphenylamine or carbazolyl structures in one molecule, where these structures are linked to each other via a single bond or a divalent group that does not contain a heteroatom; and the like can be used. A film may be formed using one of these materials alone, but a film can also be formed using a mixture of two or more of these materials. Either type of film can be used as a single layer. In addition, a stacked layer structure in which films each formed using one of these materials alone are stacked as layers, a stacked layer structure in which films each formed using a mixture are stacked as layers, or a stacked layer structure in which a film formed using one of these materials alone and a film formed using a mixture are stacked as layers may be adopted. Furthermore, a coating type polymer material such as poly(3,4-ethylenedioxythiophene) (PEDOT)/poly(styrenesulfonate) (PSS) can be used as a material for a hole injection and transport layer. With these materials, a thin film can be formed using known methods, such as vapor deposition, spin coating, and inkjet printing.

Furthermore, a material that is obtained by further p-doping a material normally used for a hole injection layer or a hole transport layer with trisbromophenylamine hexachloroantimony, a radialene derivative (see Patent Literature 6, for example), or the like is preferably used for the hole injection layer or the hole transport layer. Alternatively, a polymer compound that contains the structure of a benzidine derivative, such as TPD, in a partial structure thereof, or the like can also be used.

As a material for the electron blocking layer of the organic EL element of the present invention, compounds having an electron blocking effect can be used, examples of which include carbazole derivatives such as 4,4',4''-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-ylphenyl)adamantane (Ad-Cz); and compounds that have a triphenylsilyl group and a triarylamine structure and are typified by 9-[4-(carbazol-9-yl)phenyl]-9-[4-(triphenylsilyl)phenyl]-9H-fluorene. These materials may also serve as a material for the hole transport layer. A film may be formed using one of these materials alone, but a film can also be formed using a mixture of two or more of these materials. Either type of film can be used as a single layer. In addition, a stacked layer structure in which films each formed using one of these materials alone are stacked as layers, a stacked layer structure in which films each formed using a mixture are stacked as layers, or a stacked layer structure in which a film formed using one of these materials alone and a film formed using a mixture are stacked as layers may be adopted. With these materials, a thin film can be formed using known methods, such as vapor deposition, spin coating, and inkjet printing.

As a material for the light emitting layer of the organic EL element of the present invention, in addition to the arylamine compound of the present invention being used, metal complexes of quinolinol derivatives, including tris(8-quinolinolato)aluminum (Alq₃); various types of metal complexes; an anthracene derivative; a bisstyrylbenzene derivative; a pyrene derivative; an oxazole derivative; a poly(p-phenylene vinylene) derivative; and the like can be used. The light emitting layer may also be formed using a host material and a dopant material. As the host material, an anthracene derivative is preferably used; however, in addition to the above-described light emitting materials including the arylamine compound of the present invention, a heterocyclic compound containing an indole ring as a partial structure of a fused ring; a heterocyclic compound containing a carbazole ring as a partial structure of a fused ring; a carbazole derivative; a thiazole derivative; a benzimidazole derivative; a polydialkylfluorene derivative; and the like can be used. As the dopant material, quinacridone, coumarin, rubrene, perylene, and derivatives thereof; a benzopyran derivative; a rhodamine derivative; an aminostyryl derivative; and the like can be used. A film may be formed using one of these materials alone, but a film can also be formed using a mixture of two or more of these materials. Either type of film can be used as a single layer. In addition, a stacked layer structure in which films each formed using one of these materials alone are stacked as layers, a stacked layer structure in which films each formed using a mixture are stacked as layers, or a stacked layer structure in which a film formed using one of these materials alone and a film formed using a mixture are stacked as layers may be adopted. With these materials, a thin film can be formed using known methods, such as vapor deposition, spin coating, and inkjet printing.

Furthermore, a phosphorescent emitter can also be used as a light emitting material. As the phosphorescent emitter, a phosphorescent emitter of a metal complex of iridium, platinum, or the like can be used. Examples thereof include a green phosphorescent emitter such as Ir(ppy)₃, a blue phosphorescent emitter such as FIrpic or FIr6, and a red phosphorescent emitter such as Btp₂Ir(acac). As the host material in this case, carbazole derivatives such as 4,4'-di(N-carbazolyl)biphenyl (CBP), TCTA, and mCP, as well as the arylamine compound of the present invention, can be used as a host material with hole injectability and transportability, and p-bis(triphenylsilyl)benzene (UGH2), 2,2',2"-(1,3,5-phenylene)-tris(1-phenyl-1H-benzimidazole) (TPBI), and the like can be used as a host material with electron transportability. A high-performance organic EL element can be produced by using such materials.

To avoid concentration quenching, the doping of the host material with a phosphorescent light emitting material is preferably performed by co-deposition, with the amount of the phosphorescent light emitting material set within a range of 1 to 30 wt% with respect to the entire light emitting layer.

Materials that emit delayed fluorescence, such as CDCB derivatives such as PIC-TRZ, CC2TA, PXZ-TRZ, and 4CzIPN, can also be used as the light emitting material (see Non-Patent Literature 3, for example). With these materials, a thin film can be formed using known methods, such as vapor deposition, spin coating, and inkjet printing.

As a material for the hole blocking layer of the organic EL element of the present invention, compounds having a hole blocking effect can be used, examples of which include a phenanthroline derivative such as bathocuproine (BCP); metal complexes of quinolinol derivatives, such as bis(2-methyl-8-quinolinolate)-4-(phenylphenolato)aluminum (BAlq); various types of rare-earth complexes; an oxadiazole derivative; a triazole derivative; a triazine derivative; and the like. These materials may also serve as a material for the electron transport layer. A film may be formed using one of these materials alone, but a film can also be formed using a mixture of two or more of these materials. Either type of film can be used as a single layer. In addition, a stacked layer structure in which films each formed using one of these materials alone are stacked as layers, a stacked layer structure in which films each formed using a mixture are stacked as layers, or a stacked layer structure in which a film formed using one of these materials alone and a film formed using a mixture are stacked as layers may be adopted. With these materials, a thin film can be formed using known methods, such as vapor deposition, spin coating, and inkjet printing.

As a material for the electron transport layer of the organic EL element of the present invention, a benzimidazole derivative, an anthracene derivative, a pyrimidine derivative, and a triazine derivative are preferably used. In addition to these derivatives, metal complexes of quinolinol derivatives, such as Alq3 and BAlq, various types of metal complexes, a triazole derivative, an oxadiazole derivative, a pyridine derivative, a thiadiazole derivative, a carbodiimide derivative, a quinoxaline derivative, a pyridoindole derivative, a phenanthroline derivative, a silole derivative, and the like can be used. A film may be formed using one of these materials alone, but a film can also be formed using a mixture of two or more of these materials. Either type of film can be used as a single layer. In addition, a stacked layer structure in which films each formed using one of these materials alone are stacked as layers, a stacked layer structure in which films each formed using a mixture are stacked as layers, or a stacked layer structure in which a film formed using one of these materials alone and a film formed using a mixture are stacked as layers may be adopted. With these materials, a thin film can be formed using known methods, such as vapor deposition, spin coating, and inkjet printing.

As a material for the electron injection layer of the organic EL element of the present invention, alkali metal salts such as lithium fluoride and cesium fluoride; alkaline earth metal salts such as magnesium fluoride; metal complexes of quinolinol derivatives, such as lithium quinolinol; metal oxides such as aluminum oxide; metals such as ytterbium (Yb), samarium (Sm), calcium (Ca), strontium (Sr), and cesium (Cs); and the like can be used. The electron injection layer can be omitted by suitably selecting an electron transport layer and a cathode.

Furthermore, materials obtained by n-doping materials normally used for an electron injection layer and an electron transport layer with a metal such as cesium can be used for the electron injection layer and the electron transport layer.

For the cathode of the organic EL element of the present invention, metals with a low work function, such as aluminum, and alloys with an even lower work function, such as a magnesium-silver alloy, a magnesium-indium alloy, and an aluminum-magnesium alloy, are used as electrode materials.

As a material for the capping layer of the organic EL element of the present invention, an arylamine derivative containing a carbazolyl group, a benzoxazolyl group, or the like as a substituent, an aryldiamine derivative, a carbazole derivative, and the like can be used. A film may be formed using one of these materials alone, but a film can also be formed using a mixture of two or more of these materials. Either type of film can be used as a single layer. In addition, a stacked layer structure in which films each formed using one of these materials alone are stacked as layers, a stacked layer structure in which films each formed using a mixture are stacked as layers, or a stacked layer structure in which a film formed using one of these materials alone and a film formed using a mixture are stacked as layers may be adopted. With these materials, a thin film can be formed using known methods, such as vapor deposition, spin coating, and inkjet printing.

### Examples

Hereinafter, embodiments of the present invention will be described in greater detail using examples, but the present invention is not limited to the examples below and includes other matter that does not depart from the gist of the present invention.

### Example 1

### Synthesis of N-(2'-(9H-carbazol-9-yl)-[1,1'-biphenyl]-4-yl)-N-([1,1'-biphenyl]-4-yl)-[1,1':2',1":4",1" '-quaterphenyl]-5'-amine (Compound (59))

12.0 g of N-([1,1'-biphenyl]-4-yl)-[1,1':2',1":4",1"'-quaterphenyl]-5'-amine, 9.9 g of 9-(4'-chloro-[1,1'-biphenyl]-2-yl)-9H-carbazole, 0.5 g of tris(dibenzylideneacetone)palladium(0), 0.2 g of tri-tert-butylphosphine, and 2.9 g of sodium tert-butoxide were placed in a nitrogen-purged reaction vessel and stirred in a xylene solvent overnight under reflux. After confirming the completion of the reaction, filtration was performed, and the filtrate was concentrated to obtain a crude product. The obtained crude product was purified through recrystallization using a toluene solvent to obtain 14.5 g (yield: 72.3%) of a white powder of N-(2'-(9H-carbazol-9-yl)-[1,1'-biphenyl]-4-yl)-N-([1,1'-biphenyl]-4-yl)-[1,1':2',1":4",1" '-quaterphenyl]-5'-amine (Compound (59)).

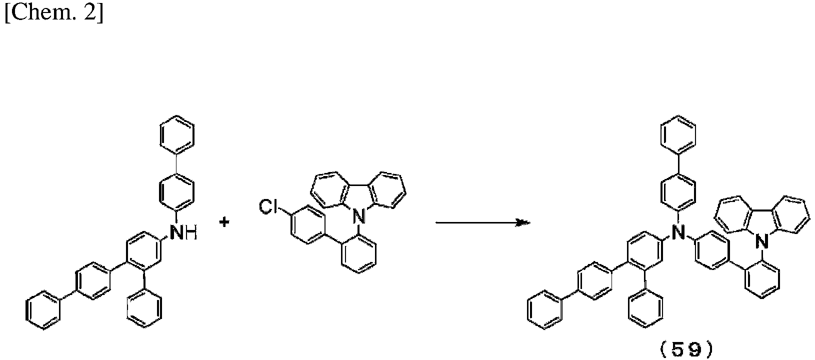

The structure of the obtained white powder was identified using NMR.

In ¹H-NMR (DMSO-d₆), the following 42 hydrogen signals were detected.
δ (ppm) = 8.13 (2H), 7.75 (1H), 7.71-7.57 (7H), 7.50 (4H), 7.43 (4H), 7.33 (2H), 7.31-7.15 (8H), 7.13 (2H), 7.02 (4H), 6.80 (5H), 6.69 (1H), 6.65 (2H).

### Example 2

### Synthesis of N-{3'-(9H-carbazol-9-yl)-[1,1'-biphenyl]-4-yl}-N-([1,1'-biphenyl]-4-yl)-[1,1':2',1":4",1" '-quaterphenyl]-5'-amine (Compound (151))

15.0 g of N-([1,1'-biphenyl]-4-yl)-[1,1':2',1":4",1"'-quaterphenyl]-5'-amine, 12.3 g of 9-(4'-chloro-[1,1'-biphenyl]-3-yl)-9H-carbazole, 0.6 g of tris(dibenzylideneacetone)palladium(0), 0.3 g of tri-tert-butylphosphine, and 3.7 g of sodium tert-butoxide were placed in a nitrogen-purged reaction vessel and stirred in a xylene solvent overnight under reflux. After confirming the completion of the reaction, filtration was performed, and the filtrate was concentrated to obtain a crude product. The obtained crude product was purified through crystallization using a toluene/acetone solvent mixture to obtain 17.3 g (yield: 69.1%) of a white powder of N-{3'-(9H-carbazol-9-yl)-[1,1'-biphenyl]-4-yl}-N-([1,1'-biphenyl]-4-yl)-[1,1':2',1":4",1" '-quaterphenyl]-5'-amine (Compound (151)).

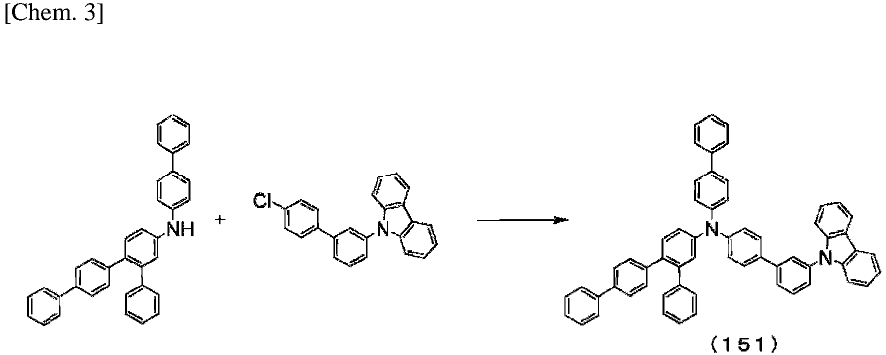

The structure of the obtained white powder was identified using NMR.

In ¹H-NMR (DMSO-d₆), the following 42 hydrogen signals were detected.
δ (ppm) = 8.26 (2H), 7.84 (1H), 7.82 (1H), 7.75 (2H), 7.73 (1H), 7.64 (6H), 7.57 (1H), 7.53 (2H), 7.48-7.38 (9H), 7.37-7.27 (4H), 7.26-7.12 (10H), 7.10 (3H).

### Example 3

### Synthesis of N-(3'-(9H-carbazol-9-yl)-[1,1'-biphenyl]-4-yl)-N-([1,1':4',1"-terphenyl]-4-yl)-[1,1':2',1" -terphenyl]-4'-amine (Compound (183))

10.0 g of N-([1,1':4',1"-terphenyl]-4-yl)-[1,1':2',1"-terpheny1]-4'-amine, 8.2 g of 9-(4'-chloro-[1,1'-biphenyl]-3-yl)-9H-carbazole, 0.4 g of tris(dibenzylideneacetone)palladium(0), 0.2 g of tri-tert-butylphosphine, and 3.0 g of sodium tert-butoxide were placed in a nitrogen-purged reaction vessel and stirred in a xylene solvent overnight under reflux. After confirming the completion of the reaction, filtration was performed, and the filtrate was concentrated to obtain a crude product. The obtained crude product was purified through recrystallization using a toluene solvent to obtain 15.1 g (yield: 90.4%) of a white powder of N-(3'-(9H-carbazol-9-yl)-[1,1'-biphenyl]-4-yl)-N-([1,1':4',1"-terphenyl]-4-yl)-[1,1':2',1" -terphenyl]-4'-amine (Compound (183)).

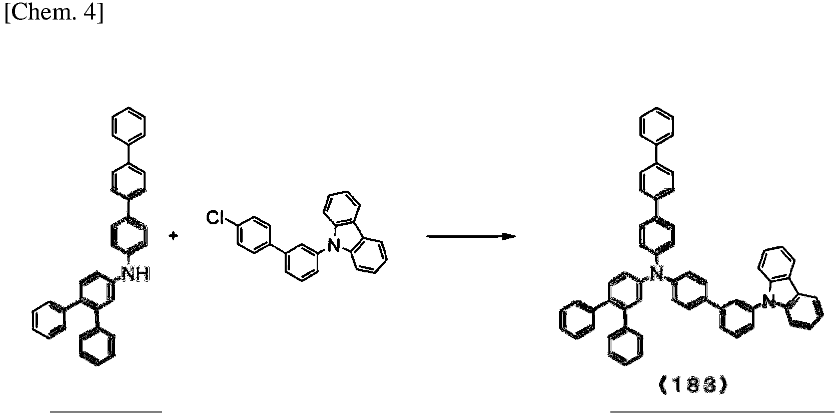

The structure of the obtained white powder was identified using NMR.

In ¹H-NMR (DMSO-d₆), the following 42 hydrogen signals were detected.
δ (ppm) = 8.26 (2H), 7.84 (1H), 7.81 (1H), 7.76-7.66 (11H), 7.56 (1H), 7.47 (2H), 7.42 (4H), 7.37 (2H), 7.29 (2H), 7.25-7.11 (11H), 7.09 (3H), 7.04 (2H).

### Example 4

### Synthesis of N-(3'-(9H-carbazol-9-yl)-[1,1'-biphenyl]-4-yl)-N-(1-(naphthalen-1-yl)phenyl-4-yl)-[1,1': 2',1"-terphenyl]-4'-amine (Compound (153))

10.0 g of N-[3'-(9H-carbazol-9-yl)-[1,1'-biphenyl]-4-yl]-[1,1':2',1"-terphenyl]-4'-amine, 5.5 g of 1-(4-bromophenyl)naphthalene, 3.4 g of sodium tert-butoxide, 0.3 g of tris(dibenzylideneacetone)palladium(0), 0.1 g of tri-tert-butylphosphine, and 100 ml of xylene were placed in a reaction vessel and stirred while being heated under reflux. After confirming the completion of the reaction, the reaction mixture was separated by column chromatography (carrier: silica gel, eluent: dichloromethane/n-heptane). The obtained crude product was purified through crystallization using a dichloromethane/n-heptane solvent mixture to obtain 7.9 g (yield: 58.1%) of a white powder of N-(3'-(9H-carbazol-9-yl)-[1,1'-biphenyl]-4-yl)-N-(1-(naphthalen-1-yl)phenyl-4-yl)-[1,1': 2',1"-terphenyl]-4'-amine (Compound (153)).

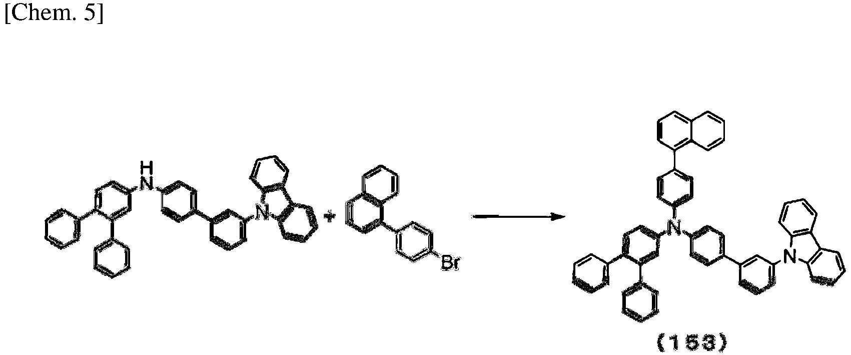

The structure of the obtained white powder was identified using NMR.

In ¹H-NMR (CDCl₃), the following 40 hydrogen signals were detected.
δ (ppm) = 8.20 (2H), 8.07 (1H), 7.94 (1H), 7.89-7.85 (2H), 7.75-7.68 (2H), 7.64 (2H), 7.58-7.14 (30H).

### Example 5

### Synthesis of N-(3'-(9H-carbazol-9-yl)-[1,1'-biphenyl]-4-yl)-N-(1-(naphthalen-2-yl)phenyl-4-yl)-[1,1': 2',1"-terphenyl]-4'-amine (Compound (163))

8.6 g of N-[3'-(9H-carbazol-9-yl)[1,1'-biphenyl]-4-yl]-[1,1':2',1"-terphenyl]-4'-amine, 4.8 g of 2-(4-bromophenyl)naphthalene, 2.9 g of sodium tert-butoxide, 0.3 g of tris(dibenzylideneacetone)palladium(0), 0.1 g of tri-tert-butylphosphine, and 86 ml of xylene were placed in a reaction vessel and stirred while being heated under reflux. After confirming the completion of the reaction, methanol was added, and the resulting precipitate was collected by filtration to obtain a crude product. The obtained crude product was purified through crystallization using a tetrahydrofuran/ethyl acetate solvent to obtain 5.0 g (yield: 42.8%) of a white powder of N-(3'-(9H-carbazol-9-yl)-[1,1'-biphenyl]-4-yl)-N-(1-(naphthalen-2-yl)phenyl-4-yl)-[1,1': 2',1"-terphenyl]-4'-amine (Compound (163)).

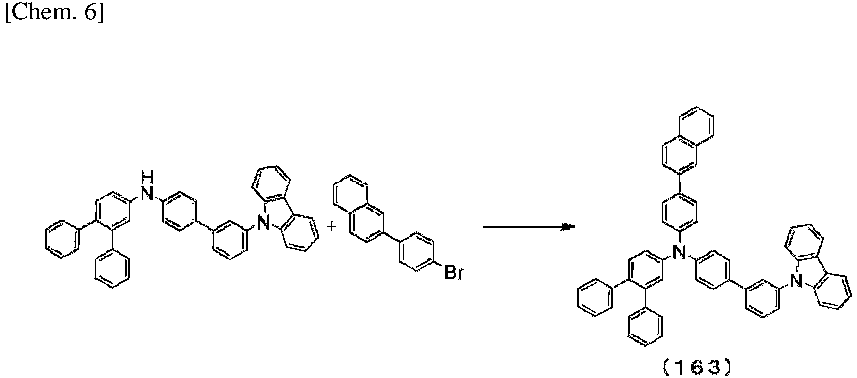

The structure of the obtained white powder was identified using NMR.

In ¹H-NMR (CDCl₃), the following 40 hydrogen signals were detected.
δ (ppm) = 8.20 (2H), 8.07 (1H), 7.95-7.88 (3H),7.84 (1H), 7.79 (1H), 7.75-7.68 (4H), 7.62 (2H), 7.56-7.44 (7H), 7.40-7.13 (19H).

### Example 6

### Synthesis of N-(3'-(9H-carbazol-9-yl)-[1,1'-biphenyl]-4-yl)-N-(dibenzofuran-4-yl)-[1,1':2',1"-terphen yl]-4'-amine (Compound (184))

15.0 g of N-[dibenzofuran-4-yl]-[1,1':2',1"-terphenyl]-4'-amine, 14.2 g of 9-(4'-chloro[1,1'-biphenyl]-3-yl-9H-carbazole, 5.3 g of sodium tert-butoxide, 0.7 g of tris(dibenzylideneacetone)palladium(0), 0.3 g of tri-tert-butylphosphine, and 150 ml of xylene were placed in a reaction vessel and stirred while being heated under reflux. After confirming the completion of the reaction, the reaction mixture was dissolved in heated toluene, silica gel was added, and the mixture was then stirred for 30 minutes and subsequently filtered through Celite. The obtained crude product was purified through crystallization using a toluene/acetone solvent mixture to obtain 6.3 g (yield: 23.7%) of a white powder of N-(3'-(9H-carbazol-9-yl)-[1,1'-biphenyl]-4-yl)-N-(dibenzofuran-4-yl)-[1,1':2',1''-terphen yl]-4'-amine (Compound (184)).

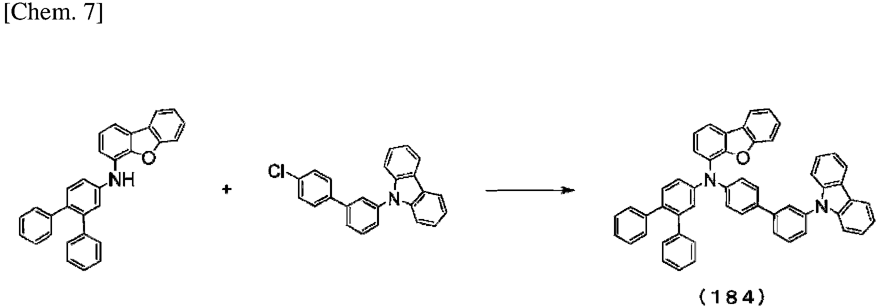

The structure of the obtained white powder was identified using NMR.

In ¹H-NMR (DMSO-d₆), the following 36 hydrogen signals were detected.
δ (ppm) = 8.26 (2H), 8.19 (1H), 8.08 (1H), 7.86 (1H), 7.83 (1H), 7.77-7.72 (3H), 7.61 (1H), 7.57 (1H), 7.51-7.40 (8H), 7.37 (1H), 7.32-7.28 (2H), 7.24-7.16 (8H), 7.13-7.00 (6H).

### Example 7

### Synthesis of N-(3'-(9H-carbazol-9-yl)-[1,1'-biphenyl]-4-yl)-N-(dibenzofuran-3-yl)-[1,1':2',1"-terphen yl]-4'-amine (Compound (185))

14.0 g of N-[dibenzofuran-3-yl]-[1,1':2',1"-terphenyl]-4'-amine, 13.2 g of 9-(4'-chloro[1,1'-biphenyl]-3-yl-9H-carbazole, 4.9 g of sodium tert-butoxide, 0.6 g of tris(dibenzylideneacetone)palladium(0), 0.3 g of tri-tert-butylphosphine, and 140 ml of xylene were placed in a reaction vessel and stirred while being heated under reflux. After confirming the completion of the reaction, the reaction mixture was dissolved in heated toluene, silica gel was added, and the mixture was then stirred for 30 minutes and subsequently filtered through Celite. The obtained crude product was purified through crystallization using a toluene/acetone solvent mixture to obtain 18.6 g (yield: 75.0%) of a white powder of N-(3'-(9H-carbazol-9-yl)-[1,1'-biphenyl]-4-yl)-N-(dibenzofuran-3-yl)-[1,1':2',1''-terphen yl]-4'-amine (Compound (185)).

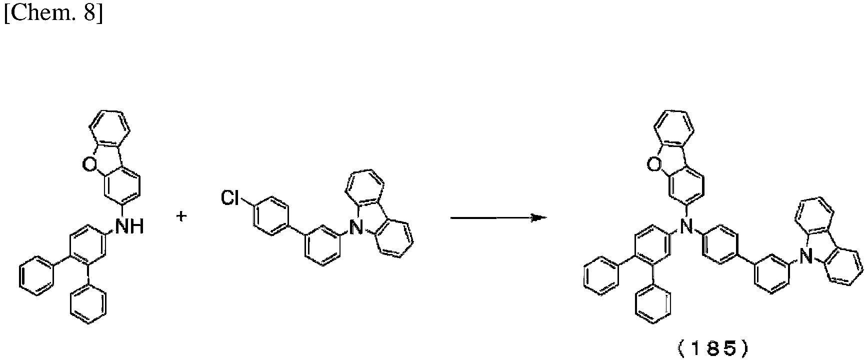

The structure of the obtained white powder was identified using NMR.

In ¹H-NMR (DMSO-d₆), the following 36 hydrogen signals were detected.
δ (ppm) = 8.27 (2H), 8.07 (2H), 7.89 (1H), 7.86 (1H), 7.81-7.74 (3H), 7.65 (1H), 7.59 (1H), 7.49-7.37 (8H), 7.33-7.17 (12H), 7.13-7.11 (3H), 7.07-7.05 (2H).

### Example 8

### Synthesis of N-(3'-(9H-carbazol-9-yl)-[1,1'-biphenyl]-4-yl)-N-(dibenzofuran-2-yl)-[1,1':2',1''-terphen yl]-4'-amine (Compound (186))

10.0 g of N-[3'-(9H-carbazol-9-yl)-[1,1'-biphenyl]-4-yl]-[1,1':2',1"-terphenyl]-4'-amine, 4.8 g of 2-bromodibenzofuran, 3.4 g of sodium tert-butoxide, 0.3 g of tris(dibenzylideneacetone)palladium(0), 0.1 g of tri-tert-butylphosphine, and 100 ml of xylene were placed in a reaction vessel and stirred while being heated under reflux. After confirming the completion of the reaction, methanol was added, and the resulting precipitate was collected by filtration to obtain a crude product. The obtained crude product was purified through crystallization using a toluene solvent to obtain 9.0 g (yield: 69.5%) of a white powder of N-(3'-(9H-carbazol-9-yl)-[1,1'-biphenyl]-4-yl)-N-(dibenzofuran-2-yl)-[1,1':2',1''-terphen yl]-4'-amine (Compound (186)).

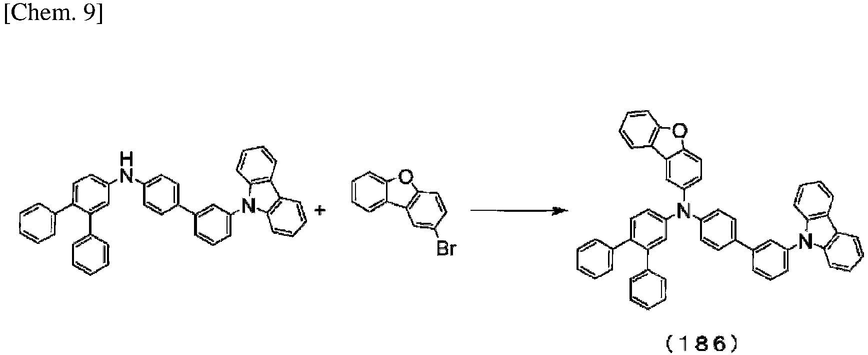

The structure of the obtained white powder was identified using NMR.

In ¹H-NMR (DMSO-d₆), the following 36 hydrogen signals were detected.
δ (ppm) = 8.27 (2H), 8.18 (1H), 8.14 (1H), 7.86 (1H), 7.82 (1H), 7.78-7.70 (5H), 7.59-7.51 (2H), 7.46-7.35 (7H), 7.32-7.28 (2H), 7.24-7.02 (14H).

### Example 9

### Synthesis of N-(3'-(9H-carbazol-9-yl)-[1,1'-biphenyl]-4-yl)-N-(dibenzofuran-1-yl)-[1,1':2',1"-terphen yl]-4'-amine (Compound (187))

10.0 g of N-[3'-(9H-carbazol-9-yl)-[1,1'-biphenyl]-4-yl]-[1,1':2',1"-terphenyl]-4'-amine, 4.8 g of 1-bromodibenzofuran, 3.4 g of sodium tert-butoxide, 0.3 g of tris(dibenzylideneacetone)palladium(0), 0.1 g of tri-tert-butylphosphine, and 100 ml of xylene were placed in a reaction vessel and stirred while being heated under reflux. After confirming the completion of the reaction, the reaction mixture was separated by column chromatography (carrier: silica gel, eluent: dichloromethane/n-heptane) to obtain 9.0 g (yield: 69.5%) of a white powder of N-(3'-(9H-carbazol-9-yl)-[1,1'-biphenyl]-4-yl)-N-(dibenzofuran-1-yl)-[1,1':2',1''-terphen yl]-4'-amine (Compound (187)).

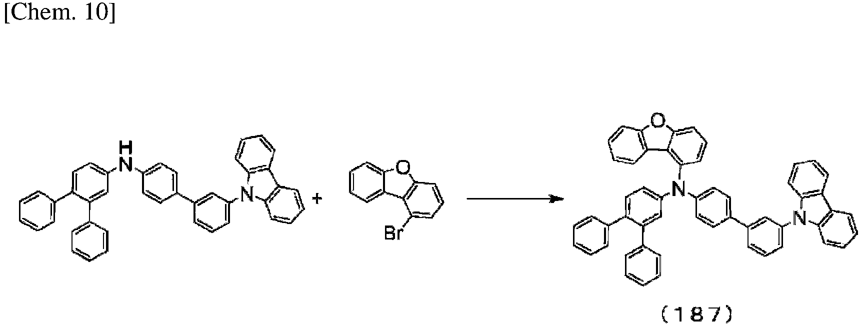

The structure of the obtained white powder was identified using NMR.

In ¹H-NMR (DMSO-d₆), the following 36 hydrogen signals were detected.
δ (ppm) = 8.26 (2H), 7.83 (1H), 7.77 (1H), 7.74-7.67 (5H), 7.63 (1H), 7.66 (1H), 7.49-7.40 (6H), 7.35 (1H), 7.30 (2H), 7.26-7.05 (14H), 6.96 (2H).

### Example 10

### Synthesis of N-(1-(9H-carbazol-9-yl)dibenzofuran-7-yl)-N-([1,1'-biphenyl]-4-yl)-[1,1':2',1":4",1‴-qu aterphenyl]-5'-amine (Compound (246))

10.2 g of N-[1,1'-biphenyl]-4-yl-[1,1':2',1":4",1‴-quaterphenyl]-5'-amine, 7.9 g of 9-(7-chloro-1-dibenzofuranyl)-9H-carbazole, 4.1 g of sodium tert-butoxide, 0.4 g of tris(dibenzylideneacetone)palladium(0), 0.2 g of tri-tert-butylphosphine, and 102 ml of xylene were placed in a reaction vessel and stirred while being heated under reflux. After confirming the completion of the reaction, methanol was added, and the resulting precipitate was collected by filtration to obtain a crude product. The obtained crude product was subjected to acetone dispersion cleaning to obtain 12.0 g (yield: 69.2%) of a white powder of N-(1-(9H-carbazol-9-yl)dibenzofuran-7-yl)-N-([1,1'-biphenyl]-4-yl)-[1,1':2',1":4",1‴-qu aterphenyl]-5'-amine (Compound (246)).

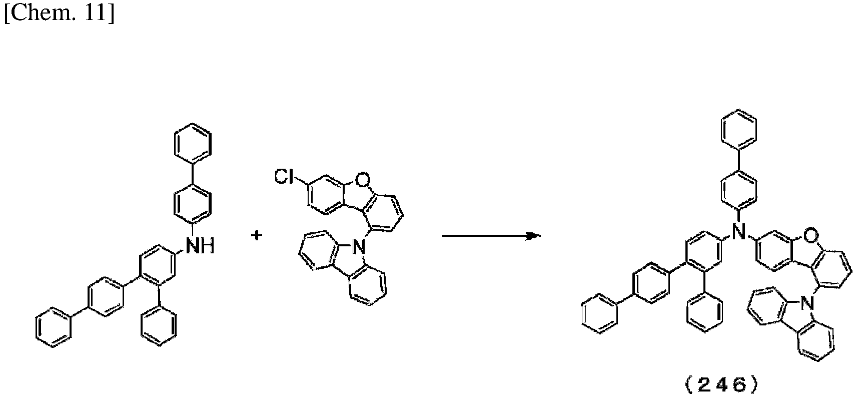

The structure of the obtained white powder was identified using NMR.

In ¹H-NMR (CDCl₃), the following 40 hydrogen signals were detected.
δ (ppm) = 8.24 (2H), 7.71 (1H), 7.64-7.57 (5H), 7.52-7.32 (17H), 7.25-7.09 (13H), 6.78 (1H), 6.28 (1H).

### Example 11

### Synthesis of N-(1-(9H-carbazol-9-yl)dibenzofuran-7-yl)-N-([1,1':4',1"-terphenyl]-4-yl)-[1,1':2',1"-ter phenyl]-4'-amine (Compound (256))

9.0 g of N-[1,1':4',1"-terphenyl]-4-yl-[1,1':2',1"-terphenyl]-4'-amine, 7.0 g of 9-(7-chloro-1-dibenzofuranyl)-9H-carbazole, 3.7 g of sodium tert-butoxide, 0.4 g of tris(dibenzylideneacetone)palladium(0), 0.2 g of tri-tert-butylphosphine, and 90 ml of xylene were placed in a reaction vessel and stirred while being heated under reflux. After confirming the completion of the reaction, methanol was added, and the resulting precipitate was collected by filtration to obtain a crude product. The obtained crude product was purified through crystallization using a tetrahydrofuran/ethyl acetate solvent to obtain 10.0 g (yield: 65.4%) of a white powder of N-(1-(9H-carbazol-9-yl)dibenzofuran-7-yl)-N-([1,1':4',1"-terphenyl]-4-yl)[1,1':2',1"-terp henyl]-4'-amine (Compound (256)).

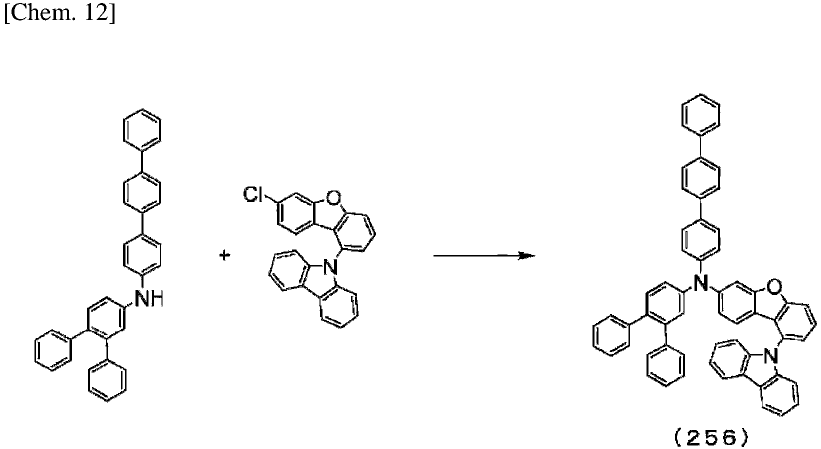

The structure of the obtained white powder was identified using NMR.

In ¹H-NMR (CDCl₃), the following 40 hydrogen signals were detected.
δ (ppm) = 8.24 (2H), 7.71-7.60 (8H), 7.56-7.46 (5H), 7.42-7.31 (7H), 7.25-7.05 (16H), 6.78 (1H), 6.28 (1H).

### Example 12

The melting point and glass transition point of the arylamine compounds obtained in Examples 1 to 11 above were measured using a high-sensitivity differential scanning calorimeter (DSC3100SA manufactured by Bruker AXS K.K.). The measurement results are organized in Table 1.

**[Table 1]**

| | Melting point (°C) | Glass transition point (°C) |
|---|---|---|
| Compound (59) | - | 129 |
| Compound (151) | - | 132 |
| Compound (183) | - | 129 |
| Compound (153) | - | 125 |
| Compound (163) | - | 125 |
| Compound (184) | - | 126 |
| Compound (185) | - | 126 |
| Compound (186) | - | 128 |
| Compound (187) | - | 128 |
| Compound (246) | - | 146 |
| Compound (256) | - | 115 |

As shown in Table 1, the arylamine compounds obtained in Examples 1 to 11 exhibited a glass transition point of 100°C or higher. These results indicate that an arylamine compound represented by the general formula (A) of the present invention is stable in a thin film state.

### Example 13

Vapor-deposited films with a film thickness of 100 nm were formed on ITO substrates using the arylamine compounds obtained in Examples 1 to 11 above, and the work function (unit: eV) was measured using an ionization potential measuring device (PYS-202 manufactured by Sumitomo Heavy Industries, Ltd.). The measurement results are organized in Table 2.

**[Table 2]**

| | Work function (eV) |
|---|---|
| Compound (59) | 5.72 |
| Compound (151) | 5.69 |
| Compound (183) | 5.65 |
| Compound (153) | 5.64 |
| Compound (163) | 5.67 |
| Compound (184) | 5.68 |
| Compound (185) | 5.73 |
| Compound (186) | 5.81 |
| Compound (187) | 5.77 |
| Compound (246) | 5.69 |
| Compound (256) | 5.72 |

As shown in Table 2, the arylamine compounds obtained in Examples 1 to 11 exhibited a more suitable energy level compared with the work function of 5.4 eV for common hole transport materials such as NPD and TPD. This indicates that an arylamine compound represented by the general formula (A) of the present invention has good hole transport capability and excellent electron blocking capability.

### Example 14

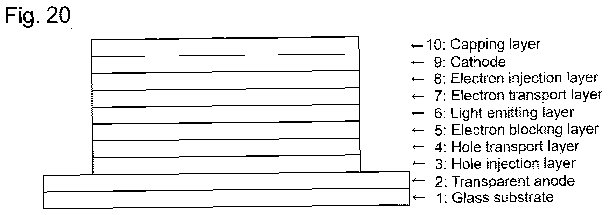

Organic EL elements were prepared in the following manner using the arylamine compounds obtained in Examples 1 to 11 above. As shown in Fig. 20, a reflective ITO electrode serving as a transparent anode 2 was formed on a glass substrate 1 in advance, and a hole injection layer 3, a hole transport layer 4, an electron blocking layer 5, a light emitting layer 6, an electron transport layer 7, an electron injection layer 8, a cathode 9, and a capping layer 10 were formed in this order on the ITO electrode by vapor deposition.

Specifically, the transparent anode 2 was formed on the glass substrate 1 by sequentially depositing an ITO film with a film thickness of 50 nm, a silver alloy reflective film with a film thickness of 100 nm, and an ITO film with a film thickness of 5 nm. After ultrasonic cleaning in isopropyl alcohol for 20 minutes, the substrate with ITO was dried for 10 minutes on a hot plate heated to 250°C. After that, UV/ozone treatment was performed for 2 minutes. Then, the glass substrate with ITO was attached inside a vacuum vapor deposition machine, and the pressure was reduced to 0.001 Pa or less.

Subsequently, the hole injection layer 3 with a film thickness of 10 nm was formed to cover the transparent anode 2 by performing binary vapor deposition of an electron acceptor (Acceptor-1) having the structural formula below and a compound (HTM-1) having the structural formula below at such vapor deposition rates that the ratio of the vapor deposition rate of Acceptor-1 to the vapor deposition rate of the compound (HTM-1) was 3:97.

A film of the compound (HTM-1) having the structural formula below was formed on the hole injection layer 3 as the hole transport layer 4 with a film thickness of 140 nm.

A film of Compound (59) of Example 1 was formed on the hole transport layer 4 as the electron blocking layer 5 with a film thickness of 5 nm.

The light emitting layer 6 with a film thickness of 20 nm was formed on the electron blocking layer 5 by performing binary vapor deposition of a compound (EMD-1) having the structural formula below and a compound (EMH-1) having the structural formula below at such vapor deposition rates that the ratio of the vapor deposition rate of the compound (EMD-1) to the vapor deposition rate of the compound (EMH-1) was 5:95.

The electron transport layer 7 with a film thickness of 30 nm was formed on the light emitting layer 6 by performing binary vapor deposition of a compound (ETM-1) having the structural formula below and a compound (ETM-2) having the structural formula below at such vapor deposition rates that the ratio of the vapor deposition rate of the compound (ETM-1) to the vapor deposition rate of the compound (ETM-2) was 50:50.

A film of lithium fluoride was formed on the electron transport layer 7 as the electron injection layer 8 with a film thickness of 1 nm.

A film of a magnesium-silver alloy was formed on the electron injection layer 8 as the cathode 9 with a film thickness of 12 nm.

Lastly, a film of a compound (CPL-1) having the structural formula below was formed as the capping layer 10 with a film thickness of 60 nm.

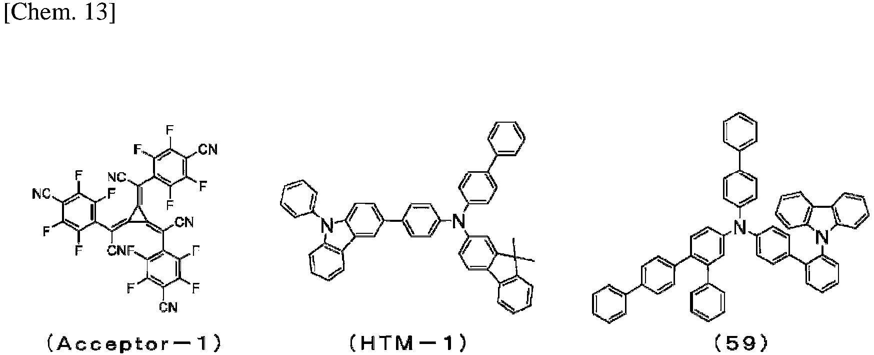

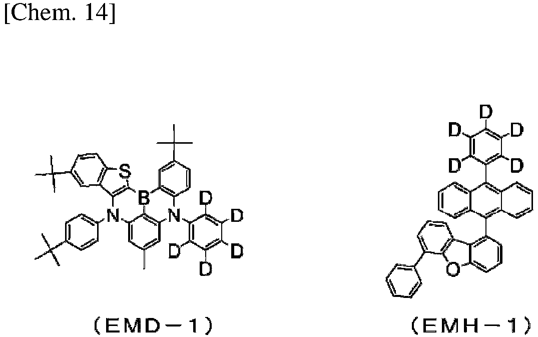

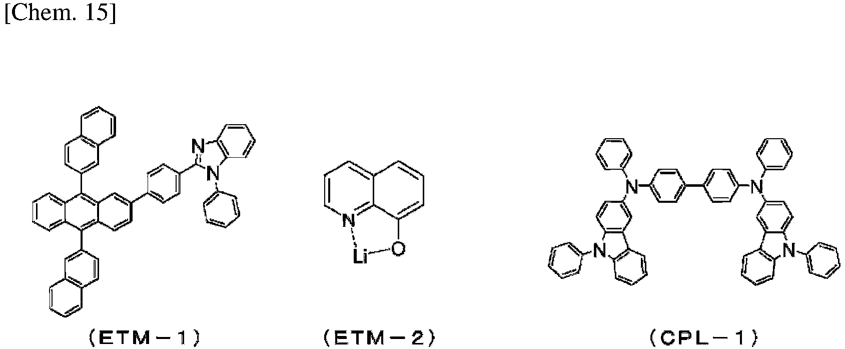

### Examples 15 to 24

Organic EL elements were prepared under similar conditions to those of Example 14, except that the compounds obtained in Examples 2 to 11, respectively, were used as the material for the electron blocking layer 5, instead of Compound (59) of Example 1.

### Comparative Example 1

For comparison, an organic EL element was prepared under similar conditions to those of Example 14, except that a compound (HTM-2) (see Patent Literature 7, for example) having the structural formula below was used as the material for the electron blocking layer 5, instead of Compound (59) of Example 1.

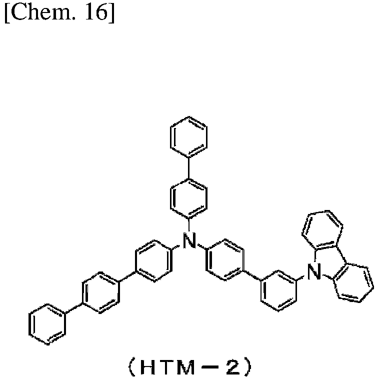

### Comparative Example 2

For comparison, an organic EL element was prepared under similar conditions to those of Example 14, except that a compound (HTM-3) (see Patent Literature 8, for example) having the structural formula below was used as the material for the electron blocking layer 5, instead of Compound (59) of Example 1.

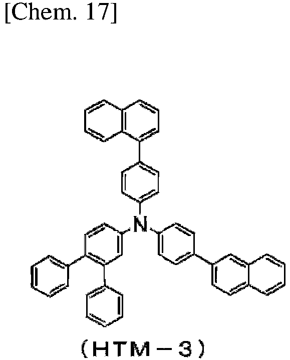

### Comparative Example 3

For comparison, an organic EL element was prepared under similar conditions to those of Example 14, except that a compound (HTM-3) (see Patent Literature 9, for example) having the structural formula below was used as the material for the electron blocking layer 5, instead of Compound (59) of Example 1.

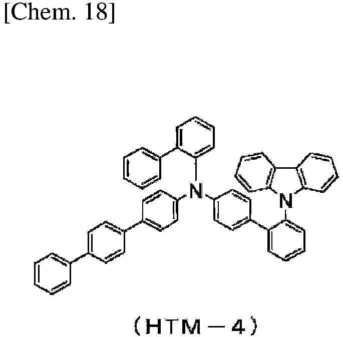

### Evaluation

With use of the organic EL elements prepared in Examples 14 to 24 and Comparative Examples 1 to 3, the voltage, luminance, light emission efficiency, and power efficiency when a current with a current density of 10 mA/cm² was applied to the elements at normal temperature in air were measured. In addition, the element lifespan was measured as follows: when constant current driving was performed with the light emission luminance (initial luminance) at the start of light emission set to 1000 cd/m², the time taken for light emission luminance to decay to 950 cd/m² (corresponding to 95% of the initial luminance being set to 100%: "decay to 95%") was measured as the element lifespan. The results are organized in Table 3.

**[Table 3]**

| | Electron blocking layer | Voltage [V] (@10 mA/cm²) | Luminance [cd/m²] (@10 mA/cm²) | Light emission efficiency [cd/A] (@10 mA/cm²) | Power efficiency [lm/W] (@10 mA/cm²) | Element lifespan decay to 95% |
|---|---|---|---|---|---|---|
| Ex. 14 | Compound (59) | 3.45 | 945 | 9.47 | 8.82 | 451 h |
| Ex. 15 | Compound (151) | 3.42 | 932 | 9.33 | 8.58 | 420 h |
| Ex. 16 | Compound (183) | 3.38 | 926 | 9.27 | 8.62 | 648 h |
| Ex. 17 | Compound (153) | 3.40 | 925 | 9.25 | 8.81 | 464 h |
| Ex. 18 | Compound (163) | 3.43 | 937 | 9.38 | 8.68 | 459 h |
| Ex. 19 | Compound (184) | 3.38 | 932 | 9.33 | 8.73 | 423 h |
| Ex. 20 | Compound (185) | 3.37 | 931 | 9.31 | 8.68 | 627 h |
| Ex. 21 | Compound (186) | 3.39 | 928 | 9.28 | 8.79 | 647 h |
| Ex. 22 | Compound (187) | 3.38 | 943 | 9.44 | 8.77 | 545 h |
| Ex. 23 | Compound (246) | 3.43 | 928 | 9.27 | 8.62 | 490 h |
| Ex. 24 | Compound (256) | 3.42 | 930 | 9.29 | 8.62 | 583 h |
| Com. Ex. 1 | HTM-2 | 3.50 | 908 | 9.11 | 8.33 | 408 h |
| Com. Ex. 2 | HTM-3 | 3.56 | 890 | 8.91 | 8.11 | 365 h |
| Com. Ex. 3 | HTM-4 | 3.53 | 869 | 8.73 | 8.29 | 386 h |

As shown in Table 3, when a current with a current density of 10 mA/cm² was applied, the light emission efficiency of the organic EL elements of Comparative Examples 1 to 3 was 8.73 to 9.11 cd/A, whereas the organic EL elements of Examples 14 to 24 exhibited a higher efficiency of 9.25 to 9.47 cd/A. In terms of power efficiency as well, the organic EL elements of Comparative Examples 1 to 3 exhibited a power efficiency of 8.11 to 8.33 lm/W, whereas the organic EL elements of Examples 14 to 24 exhibited a higher efficiency of 8.58 to 8.82 lm/W. Furthermore, in terms of element lifespan (decay to 95%), the organic EL elements of Comparative Examples 1 to 3 exhibited lifespans of 365 to 408 hours, whereas the organic EL elements of Examples 14 to 24 exhibited lifespans of 420 to 648 hours, indicating extended element lifespans.

As is clear from the above-described results, the organic EL elements of the present invention have been found to achieve higher light emission efficiency and a longer lifespan compared with conventional organic EL elements due to the use of the arylamine compounds with high hole mobility and excellent electron blocking capability.

### Industrial Applicability

An organic EL element using an arylamine compound of the present invention enables increased light emission efficiency and also improved durability of the organic EL element, and can therefore be applied to uses such as home electric appliances and lighting equipment, for example.

### List of Reference Numerals

- 1: Glass substrate
- 2: Transparent anode
- 3: Hole injection layer
- 4: Hole transport layer
- 5: Electron blocking layer
- 6: Light emitting layer
- 7: Electron transport layer
- 8: Electron injection layer
- 9: Cathode
- 10: Capping layer

## Claims

1. An arylamine compound represented by the general formula (A) below: 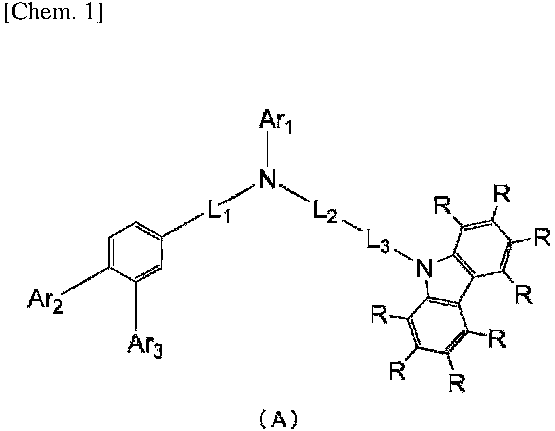
wherein Ar₁, Ar₂, and Ar₃ each independently represent a substituted or unsubstituted aromatic hydrocarbon group, a substituted or unsubstituted aromatic heterocyclic group, or a substituted or unsubstituted fused polycyclic aromatic group,
L₁, L₂, and L₃ each independently represent a single bond, a substituted or unsubstituted divalent aromatic hydrocarbon group, a substituted or unsubstituted divalent aromatic heterocyclic group, or a substituted or unsubstituted divalent fused polycyclic aromatic group, and
each R independently represents a hydrogen atom, a heavy hydrogen atom, a substituted or unsubstituted aromatic hydrocarbon group, a substituted or unsubstituted aromatic heterocyclic group, or a substituted or unsubstituted fused polycyclic aromatic group.

2. The arylamine compound according to claim 1, wherein each R in the general formula (A) independently represents a hydrogen atom, a heavy hydrogen atom, or a substituted or unsubstituted phenyl group.

3. The arylamine compound according to claim 1, wherein L₁, L₂, and L₃ in the general formula (A) each independently represent a single bond, an unsubstituted divalent aromatic hydrocarbon group, an unsubstituted divalent aromatic heterocyclic group, or an unsubstituted divalent fused polycyclic aromatic group.

4. The arylamine compound according to claim 3, wherein L₁, L₂, and L₃ in the general formula (A) each independently represent a single bond, an unsubstituted divalent phenylene group, an unsubstituted divalent naphthylene group, or an unsubstituted divalent dibenzofuranyl group.

5. The arylamine compound according to claim 4, wherein L₃ in the general formula (A) represents an unsubstituted 1,2-phenylene group, an unsubstituted 1,3-phenylene group, an unsubstituted 1,7-dibenzofuranyl group, or an unsubstituted 2,7-dibenzofuranyl group.

6. The arylamine compound according to claim 4, wherein L₁ and L₂ in the general formula (A) each independently represent a single bond or an unsubstituted 1,4-phenylene group.

7. The arylamine compound according to claim 1, wherein at least one of Ar₁, Ar₂, and Ar₃ in the general formula (A) represents a substituted or unsubstituted phenyl group.

8. An organic EL element comprising:
a pair of electrodes; and
an organic layer sandwiched between the electrodes,
wherein the organic layer contains the arylamine compound according to any one of claims 1 to 7.

9. The organic EL element according to claim 8, wherein the organic layer is a hole transport layer, an electron blocking layer, a hole injection layer, or a light emitting layer.

10. An electronic device comprising an element comprising:
a pair of electrodes; and
an organic layer sandwiched between the electrodes,
wherein the organic layer contains the arylamine compound according to any one of claims 1 to 7.
